(19) Europäisches Patentamt
European Patent Office
Office européen des brevets

(11) **EP 4 759 139 A1**

(12) **EUROPEAN PATENT APPLICATION**

(43) Date of publication:
**17.06.2026 Bulletin 2026/25**

(21) Application number: **24220250.5**

(22) Date of filing: **16.12.2024**

(51) International Patent Classification (IPC):
***A01P 7/00*** *(2006.01)* ***A61K 31/713*** *(2006.01)*
***C12N 15/11*** *(2006.01)* ***A01N 63/60*** *(2020.01)*

(52) Cooperative Patent Classification (CPC):
(C-Sets available)
**A01P 7/00; A01N 63/60; A61K 31/713;**
**C12N 15/113;** C12N 2310/14 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Fraunhofer-Gesellschaft zur Förderung der angewandten Forschung e.V.**
**80686 München (DE)**

(72) Inventors:
- **Hellmann, Christoph**
  **35392 Gießen (DE)**
- **Geisler, Pascal-Marcel**
  **35392 Gießen (DE)**
- **Lee, Kwang-Zin**
  **35392 Gießen (DE)**
- **Knorr, Eileen**
  **35392 Gießen (DE)**
- **Hardes, Kornelia**
  **35392 Gießen (DE)**
- **Oberpaul, Markus**
  **35392 Gießen (DE)**
- **Vilcinskas, Andreas**
  **35392 Gießen (DE)**

(74) Representative: **Kuttenkeuler, David**
**SKM-IP PartGmbB**
**Oberanger 45**
**80331 München (DE)**

(54) **NUCLEID ACID SURFACTANT PARTICLE COMPOSITION FOR THE CONTROL OF PEST- AND VECTOR ARTHROPODS**

(57) The invention is based on the finding, that the compositions provided provide protection to the dsRNA against environmental factors by comprising surfactant particles that encompass the dsRNA. Arthropods that feed on plants treated with a composition of the invention ingest said composition. The inventors found that the surfactant particles in the compositions of the invention can be used for the delivery of dsRNA into arthropods. In its aspects, the application pertains to said compositions, a use of said composition for the delivery of dsRNA to arthropods, and to a method of the manufacture of the compositions herein

EP 4 759 139 A1

(52) Cooperative Patent Classification (CPC): (Cont.)

C-Sets
**A01N 63/60, A01N 25/02, A01N 25/30**

## Description

### FIELD OF THE INVENTION

The invention is based on the finding, that the compositions provide protection to the dsRNA against environmental factors by comprising surfactant particles that encompass the dsRNA. Arthropods that feed on plants treated with a composition of the invention ingest said composition. The inventors found that the surfactant particles in the compositions of the invention can be used for the delivery of dsRNA into arthropods. In its aspects, the application pertains to said compositions, a use of said composition for the delivery of dsRNA to arthropods, and to a method of the manufacture of the compositions herein.

### DESCRIPTION

Arthropods, particularly insects, can represent a threat for humankind - for example, in damaging crops (agriculture) and in acting as vectors for diseases (health). Sprayable insecticides represent the most relevant technical approach to control pest insects and reduce their devasting effects on crop yields and on human health.

New developments of insecticides aim for a high selectivity to target the pest insect with minimal negative impact on non-target organisms. The technical implementation of one of the most promising new mode of action pest control strategy is based on ribonucleic acid interference (RNAi). The main advantages of RNAi mediated pest control are i) the high target-species specificity with low off-target effects and ii) the biodegradation of the active RNA molecules in the environment within a few days/weeks. The latter prevents the undesirable accumulation of pesticide residues after typical treatment periods and differentiates this approach from many conventional pesticides, due to their longer and greater stability in the environment.

Ribonucleic acid (RNA), an essential building block of all life, represents a class of bioactive molecules that offer enormous potential for the development of sustainable and highly species-dependent plant protection products. Such RNA-based crop protection concepts are based on the natural process of RNAi, in which highly selective and active double-stranded RNA molecules (dsRNA) can lead to a downregulation of target genes and trigger lethal effects in target organisms. As a result, undesirable side effects on non-target organisms in which the targeted gene sequence is absent can be minimized or even completely avoided.

One of the largest challenges for the technical implementation of RNAi pest control products is the temporary protection of the active RNA molecules against degradation by using appropriate formulation strategies. In particular nucleases that are present in the environment and in the digestion system of arthropods lead to undesirable degradation before the active RNA molecule reaches the target location inside the organism. In scientific literature, the development of suitable RNAi formulations for plant protection and insect control is a highly active and broad field of research. In 2024, the first RNAi plant protection product was introduced to the US market to protect potato cultures against the Colorado Potato Beetle.

The sensitivity of insect pests to RNAi depends on factors like degradation of the dsRNA by RNases in the insect saliva or intestinal fluid, high gut pH and reduced uptake of dsRNA by cells, and other limiting factors. A suitable protection and delivery of the active RNA molecules to the target location have not been successful in these critical insect species yet. Improving the efficiency of RNAi is a deciding task for commercializing RNAi products in agriculture as an alternative to conventional pesticides. Developing a suitable dsRNA formulation for technically most relevant spray applications is currently one of the largest challenges in crop protection.

Most dsRNA formulations reported so far use nanoparticle carriers including polymers, lipids and minerals. In general, the formation process of such particles is controlled by ionic interactions of cationic charged substances and anionic charged nucleic acids. Cationic substances such as quaternary or ionizable ternary fatty amines or zwitterion substances such as phosphocholines are amphiphilic and consist of lipophilic and hydrophilic characteristics within their molecular structure. By self-assembly, such molecules form complex particulate structures including lipids and liposomes which can encapsulate various substances including nucleic acid.

However, the structure-property-correlation of formulations for arthropod control purposes is not yet well explored. The inventors were able to develop a surfactant particle-based double strand RNA (dsRNA) formulation, in particular a lipid nanoparticle (LNP)-based double strand RNA (dsRNA) formulation, that provides the following characteristics in optimized configuration: 1) the protection of the dsRNA against nucleases 2) stability of the formulation against non-ionic surfactants 3) RNA release of the formulation via anionic surfactant 4) At least a 2 months shelf life of the nucleic acid at room temperature and under sunlight conditions 5) uptake of dsRNA LNP formulations by mosquito larva and a stability in the mosquito head region for up to 2 h post feeding 6) low cost of the formulation components using multiple-component recipes.

Thus, it is an object of the invention to provide novel compositions that can be used for the protection of dsRNA against degradation by their environment, for example against nucleases. A further object of the invention is to use the

novel compositions herein for the delivery of the dsRNA to arthropods. Specifically, an object of the invention is to use the novel compositions herein as a plant protection agent.

BRIEF DESCRIPTION OF THE INVENTION

Generally, and by way of brief description, the main aspects of the present invention can be described as follows:

In **the first aspect,** the invention pertains to a composition comprising a surfactant particle with an average hydrodynamic diameter of 20 to 200 nm when determined by dynamic light scattering, wherein the surfactant particle encompasses a double-stranded RNA (dsRNA) for gene silencing against an arthropod target gene, wherein the surfactant particle comprises

Compound A with the structure (A-I) or (A-II);

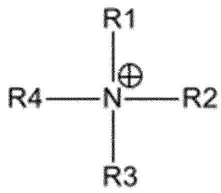

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group; or

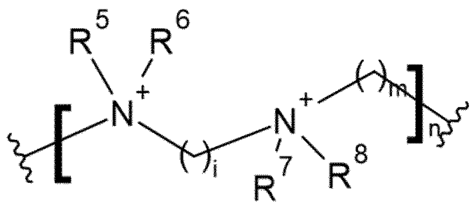

(A-II),

wherein

$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;

i and m are each independently selected from 1 to 24; and

n is 1 to 1000.

**In a second aspect,** the invention pertains to a use of a composition for the delivery of a dsRNA to an arthropod, wherein the composition comprises a surfactant particle with an average hydrodynamic diameter of 20 to 200 nm when determined by dynamic light scattering, wherein the surfactant particle encompasses a double-stranded RNA (dsRNA), wherein the surfactant particle comprises

Compound A with the structure (A-I) or (A-II);

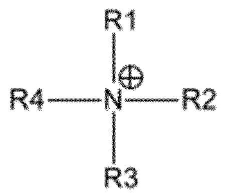

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group;

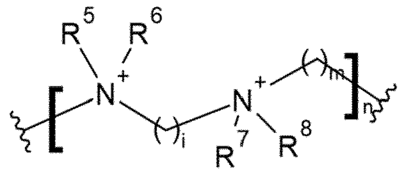

(A-II),

wherein

$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;

i and m are each independently selected from 1 to 24; and

n is 1 to 1000, and

wherein

the use comprises a step of oral ingestion of the composition by the arthropod.

**In a third aspect,** the invention pertains to a method of manufacturing a composition of the first aspect of the invention, wherein the method comprises mixing a solution 1 comprising a Compound A, a Compound B and a Compound C with a solution 2 comprising the dsRNA.

DETAILED DESCRIPTION OF THE INVENTION

In the following, the elements of the invention will be described. These elements are listed with specific embodiments; however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

In **the first aspect,** the invention pertains to a composition, comprising a surfactant particle with an average hydrodynamic diameter of 20 to 200 nm when determined by dynamic light scattering, wherein the surfactant particle encompasses a double-stranded RNA (dsRNA) for gene silencing against an arthropod target gene, wherein the surfactant particle comprises

Compound A with the structure (A-I) or (A-II);

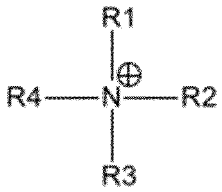

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group; or

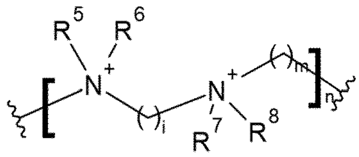

(A-II),

wherein

$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;

i and m are each independently selected from 1 to 24; and

n is 1 to 1000.

Preferably, the surfactant particle comprises a lamellar structure. In preferred embodiments, the surfactant particle is a unilamellar or a multilamellar surfactant particle. The surfactant particle can comprise a solidified or a liquid core. An unilamellar surfactant particle comprises a surfactant bilayer, a multilamellar surfactant particle comprises multiple surfactant bilayers. A "surfactant bilayer" is a unilamellar structure that comprises surfactants, wherein the hydrophilic head groups of the surfactants point away from the bilayer and the hydrophobic tail groups point towards the bilayer. Preferably, the surfactant particle is a vesicle. A such a vesicle comprises a surfactant bilayer, wherein the surfactant bilayer encloses a hydrophilic core. Preferably said hydrophilic core contains an aqueous solution. Such an aqueous solution is a solvent-system whose main fraction by molarity is water. Preferably, the surfactant particle comprises a spherical shape. Preferably, the vesicle comprises one surfactant bilayer. Preferably the surfactant particle is a micelle. The term "micelle" refers to a surfactant nanoparticle that comprises a hydrophobic core matrix that is formed from the hydrophobic tails of the surfactant molecules. Such a micelle comprises surfactants whose hydrophilic head groups point away from the micelle core, while the hydrophobic tail groups of the surfactants point to the interior of the nanoparticle. Preferably the surfactant particle is a surfactant bilayer sheet. Surfactant particles, such as vesicles or micelles can be identified by methods known in the art, e.g, by Cryo-TEM, SEM, X-ray scattering, dynamic light scattering, sedimentation field flow fractionation, optical microscopy and/or zeta potential measurement.

In preferred embodiments, the surfactant particle is a lipid nanoparticle (LNP). In preferred embodiments, the lipid nanoparticle (LNP) is a unilamellar or a multilamellar LNP. The term "lipid nanoparticle" or "LNP", as used herein, refers to a surfactant particle, wherein the surfactant comprises a lipid. Preferably, the LNP is a liposome. The term "liposome" as used herein refers to a vesicle, wherein the surfactant comprises a lipid.

Therefore, a liposome comprises a surfactant bilayer that comprises a lipid, wherein the surfactant bilayer encloses a hydrophilic core. Preferably said hydrophilic core contains an aqueous solution as defined above. Preferably, the LNP comprises a spherical shape. Preferably, the liposome comprises one surfactant bilayer.

The composition of the invention comprises a surfactant particle with an average hydrodynamic diameter of 20 to 200 nm when determined by dynamic light scattering. This average hydrodynamic diameter refers to the intensity-based average hydrodynamic diameter (Zaverage) that is obtained when an dispersion of the surfactant particle of the invention in deionized water is measured via dynamic light scattering using a Zetasizer Nano ZS (Malvern Panalytical) in a DET1070

cell (Mavern Panalytical) with the following measurement parameters: reflective index (RI) = 1.330; viscosity (cP) = 0.8872; measurement position = 5.5 mm, attenuator = 6 to 7, initial equilibration time = 0.5 min, measurement temperature 25°C; number of scans: 50; software Zetasizer v.7.10

When referenced herein, the term "Zeta-potential" refers to the average zeta-potential of the sample when measured in deionized water at pH = 7. This zeta-potential is obtained when an aqueous dispersion comprising the surfactant particle of the invention is measured using a Zetasizer Nano ZS (Malvern Panalytical) in a DET1070 cell (Mavern Panalytical) with the following measurement parameters: reflective index (RI) = 1.330; viscosity (cP) = 0.8872; measurement position = 5.5 mm, attenuator = 9 to 10, initial equilibration time = 0.5 min, measurement temperature 25°C; number of scans: 50; software Zetasizer v.7.10.

In preferred embodiments, the surfactant particle comprises a zeta potential of at least 20 mV at pH 7, or of at least 30 mV at pH 7. With an increasing absolute zeta-potential of the surfactant particles, their colloidal stability is increased.

In preferred embodiments, the composition comprises particle sizes with a polydispersity index (PDI) of 0 to 1, more preferably 0.05 to 0.8, even more preferably of preferably of 0.05 to 0.6. In preferred embodiments, the composition comprises particle sizes with a polydispersity index (PDI) of <0.6; more preferably, the composition comprises particle sizes with a polydispersity index of <0.35. The PDI is a dimensionless parameter that is calculated from the standard-deviation and mean particle size as follows:

$$PDI = \left(\frac{standard\ deviation}{mean\ particle\ size}\right)^2$$

The smaller the PDI of a sample, the higher its homogeneity. For nanoparticle-based compositions, a low PDI is considered advantageous as an increasing homogeneity in particle size also leads to an increased homogeneity in other particle properties such as amount of loading, stability and cellular uptake.

In preferred embodiments, the surfactant particle comprises a symmetry parameter (width to length ratio) of 1 or < 1, more preferably of 0.1 to 1, more preferably 0.5 to 1, most preferably 0.9 to 1.

Surfactant particles of the invention can be manufactured by mixing an organic phase comprising Compound A with an aqueous phase comprising the dsRNA. A person skilled in the art can adjust the morphology and size of the surfactant particles during this mixing process by controlling the mixing parameters.

The surfactant particle of the invention encompasses the dsRNA. In the context of the invention, the expression "wherein the surfactant particle encompasses a dsRNA" includes that the surfactant particle complexes, surrounds, and/or encapsulates the dsRNA. In this context the term "encapsulates" refers to a state, wherein the dsRNA is in a compartment that is formed by the surfactant particle. The expression "surrounds" in this context refers to a state, in which the dsRNA is embedded within a matrix that formed by the surfactant particle, for example wherein said matrix is solid or is a hydrophobic core (e.g. in the case that the surfactant particle is a micelle), or wherein the matrix is a surfactant bilayer, for example when the surfactant particle is a vesicle.

By encompassing the dsRNA, the surfactant particle of the invention protects the encompassed nucleic acid against degradation. For example, the inventors have demonstrated that the compositions improve the stability of nucleic acids such as dsRNA against degradation by nucleases (with a stability for at least 30 min) and against various pH values ranging from pH 3 up to pH 11 (with a stability for at least 24 h).

The term "alkyl" refers to a saturated straight or branched hydrocarbon group. In preferred embodiments, the alkyl group comprises 1 to 24, more preferably 1 to 18, even more preferably 1 to 12 carbon atoms. Exemplary alkyl groups include methyl, ethyl, propyl, iso-propyl (also called 2-propyl or 1-methylethyl), butyl, iso-butyl, tert-butyl, n-pentyl, iso-pentyl, sec-pentyl, neo-pentyl, 1,2-dimethyl-propyl, iso-amyl, n-hexyl, iso-hexyl, sec-hexyl, n-heptyl, iso-heptyl, n-octyl, 2-ethyl-hexyl, n-nonyl, n-decyl, n-undecyl, n-dodecyl, and the like. The term "alkenyl group" refers an unsaturated linear or branched hydrocarbon group having at least one carbon-carbon double bond. In preferred embodiments, such an alkenyl group comprises 2 to 24, more preferably 2 to 18, more preferably 2 to 12 carbon atoms. The carbon-carbon double bond(s) may be in cis (Z) or trans (E) configuration. The term "alkynyl" refers to a linear or branched hydrocarbon having at least one carbon-carbon triple bond.

In the context of the invention, if a group is "substituted", for example as in "substituted alkyl", this term indicates that a hydrogen atom that is attached to said "substituted group" is replaced by another atom or group. In this context, one, two, three or more, preferably all, hydrogen atoms can be replaced with a substituent other than hydrogen. The term "substituted" in this context does not indicate a chemical mechanism, by which the substitution takes place.

The term "substituent" indicates a first group or first atom that is bound to another second group or second atom via a chemical bond.

In structures depicted in this disclosure, the wavy symbol as in "

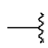

" indicates position, by which an atom or group is bound to the rest of the compound.

As used herein, the term "polynucleotide" refers to a nucleic acid molecule containing multiple nucleotides. It includes the term "oligonucleotides" that is often used to describe polynucleotides with 25 or less nucleotides.

The terms "nucleic acid" and "nucleotides" comprises deoxyribonucleotides, ribonucleotides and/or nucleotide analogues. The term "polynucleotide" therefore encompasses RNA, DNA, RNA/DNA hybrids, chemically modified polynucleotides or a mixture thereof. Such chemically modified oligonucleotides and/or polynucleotides are well known in the art; see, e. g., Verma and Eckstein (1998) Annu. Rev. Biochem., 67:99-134. Preferably, the polynucleotides is in single- or double-stranded, sense or antisense form.

Ribonucleic acid (RNA) is a polynucleotide comprising nucleotides with the nucleobases adenine (A), cytosine (C), guanine (G), and uracil (U) that are linked via a ribose-phosphate backbone. The phosphate groups of the backbone are negatively charged and have anionic character. RNA molecules show strong inter- and intramolecular interactions and can form hydrogen-bonds between the complementary bases, for example between A-U and C-G, resulting in conformations that depend on molar ratios of the respective nucleobases.

In preferred embodiments, the RNA includes non-canonical nucleotides such as inosine, thiouridine, or pseudouridine. In some aspects, the RNA includes chemically modified nucleotides. For example, the naturally occurring phosphodiester backbone of an oligonucleotide or polynucleotide can be partially or completely modified with phosphorothioate, phosphorodithioate, or methylphosphonate intemucleotide linkage modifications. Further modifications can include modified nucleoside bases or modified sugars including polynucleotides that are labeled with a fluorescent moiety (e. g., a fluorescein-based moiety or a rhodamine-based moiety) or other label (e. g. a biotin-based label).

As used herein, the term "dsRNA" includes a molecule comprising two antiparallel polynucleotide strands and a molecule comprising a polynucleotide strand forming a double stranded structure featuring a complementary region that is bound together via hydrogen bonds, and wherein at least one nucleotide strand of the molecule comprising two antiparallel polynucleotide strands and the molecule comprising a polynucleotide strand forming a double stranded structure is a ribonucleotide strand. In preferred embodiments of the invention, the two antiparallel polynucleotide strands are both ribonucleotide strands. The two antiparallel polynucleotide strands of a dsRNA can be perfectly complementary to each other or comprise one or more mismatches up to a degree where any one additional mismatch causes the disassociation of the two antiparallel strands. In preferred embodiments, the polynucleotide strand forming a double stranded structure can comprise one or more mismatches up to a degree where any one additional mismatch causes the disassociation of the double stranded structure. A dsRNA as defined herein may include nucleobase modifications or substitutions. Exemplary modified nucleobases include synthetic and natural nucleobases such as 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl anal other 8-substituted adenines and guanines, 5-halo, particularly 5-bromo, 5-trifluoromethyl and other 5-substituted uracils and cytosines, 7-methylguanine and 7-methyladenine, 8-azaguanine and 8-azaadenine, 7-deazaguanine and 7-daazaadenine and 3-deazaguanine and 3-deazaadenine.

Overall, a dsRNA as used in the context herein, can comprise secondary structures such as helices, hairpins, loops and/or combinations of single and double stranded regions. In the context herein, the dsRNA can be a double-stranded RNA formed by intramolecular hybridization, or double-stranded RNA formed by intermolecular hybridization. Preferably the dsRNA is a single-stranded RNA that self-hybridizes to form a hairpin structure having an at least partially double-stranded structure. Said hairpin structure can include at least one segment that will hybridize under physiological conditions in the cell to RNA transcribed from the gene targeted for gene silencing.

The dsRNA of the invention is for gene silencing against an arthropod target gene.

Preferably, the "arthropod target gene" is a gene that is associated with the fitness, well-being, and/or reproductive capacity of the arthropod. For example, the arthropod target gene may be one or more of a gene that is associated the arthropod morphology (for example the arthropod target gene may be associated with the establishment of anterior-posterior polarity, segmentation and appendage function and/or formation), cuticle formation, molting, chitin metabolism, circadian rhythm, metamorphosis, sex determination, reproduction, digestion, metabolism, sensory organ function, nervous system function, immune system function, response to stress, cellular integrity, cellular processes and protein transport, and inhibitors of apoptosis proteins of the arthropod.

The term "gene" refers to any portion of a nucleic acid that provides for expression of a transcript or encodes a transcript. A "gene" can include, but is not limited to, a promoter region, 5' untranslated regions, transcript encoding regions that can include intronic regions, 3' untranslated regions, or combinations of these regions. In embodiments, the target gene can include a coding or non-coding sequence or both.

The term "gene silencing", as used herein, preferably refers to a down-regulation of the expression of a target gene. Therefore, the term includes the reduction of a gene product such as a transcript and/or a polypeptide. An exemplary transcript is RNA such as mRNA. The mechanisms governing dsRNA mediated down-regulation of gene expression are still investigated. The term "gene silencing" includes a group of regulatory mechanisms mediated by RNA molecules such

as RNA interference (RNAi). In preferred embodiments, the gene silencing is RNAi.

RNAi refers to a phenomenon, induced by a dsRNA, in which target RNA is degraded in a sequence-specific manner. Without wishing to be bound by theory, one of the mechanisms of RNAi relies on cleaving the double-stranded nucleic acid molecule by Dicer protein according to its length and then, one of the strands (referred to as antisense strand or guide strand) is incorporated into RNA-induced silencing complex (RISC) containing Argonaute (AGO) protein. RISC is guided by an antisense strand (guide strand) having a sequence complementary to target RNA so that the RISC recognizes and cleaves the target RNA. When the target RNA is mRNA, the expression of the corresponding protein that is encoded by the mRNA is reduced. The antisense strand of a dsRNA molecule refers to a strand having a sequence complementary to a target sequence.

For example, a dsRNA comprising an antisense sequence with at least consecutive 16 nucleotides that is 100% complementary to a target nucleotide sequence or a double-stranded RNA comprising an antisense sequence with at 26 consecutive nucleotides that comprise an antisense sequence with two or less mismatches to the target sequence can trigger RNAi. In the case of larger RNA molecules, initial investigations revealed that an antisense sequence with a complementarity of at least 80% to a target gene, preferably a transcript thereof, can trigger RNAi effectively.

In preferred embodiments, the dsRNA is a siRNA, shRNA and/or a miRNA. These small RNA molecules are beneficial as they have the ability to transcriptionally-suppress the expression of the target genes. As used herein, the term "siRNA" (also referred to herein interchangeably as "small interfering RNA"), is a class of double-stranded RNA molecules having about 18-25 nucleotides in length. Preferably, the double-stranded siRNA comprises an antisense sequence with 100% complementarity or less than two mismatches with a target gene sequence, preferably a transcript such as an RNA thereof, preferably wherein the RNA is a mRNA.

As used herein, the term "target-specific sequence" refers to a nucleic acid sequence that is essentially complementary to a target sequence. Therefore, the target-specific sequence is an antisense sequence to the target nucleotide sequence. In some embodiments, the target nucleotide sequence is a coding region of a mRNA, a 5' untranslated region, a 3'untranslated region, an intron, a promoter, an enhancer, a terminator, an rRNA, a tRNA, a small nuclear RNA (snRNA), a small nucleolar RNA (snoRNA), a non-coding RNA involved in RNA interference, and any combination thereof. Most preferably, the target nucleotide sequence is an mRNA molecule.

Preferably, the term "complementary" as used herein indicates that the antisense sequence hybridizes under physiological conditions in the cells of the arthropod to the target gene sequence, preferably a transcript such as an RNA thereof, preferably wherein the RNA is a mRNA.

In the composition of the invention, the Compound A functions as a dsRNA complexation agent. Therefore, it is able to form a complex with dsRNA. Compound A is a cationic ammonium derivative. Preferably, Compound A is a monomeric or polymeric compound. In the compositions of the invention, said cationic complexation agent (Compound A) forms a complex with the negatively charged dsRNA. Moreover, Compound A is a surfactant. As such, it is a component of the surfactant particles in the composition of the invention. Compound A is integrated in the surfactant bilayer, more preferably the lipid bilayer of the surfactant particle. Compound A complexes and encompasses the dsRNA. This leads to a stabilization of the dsRNA, for example against a large range of pH conditions and against enzymes such as nucleases.

The inventors found that the composition of the invention can be used for plant protection. Arthropods, in particular insects, that feed on plants treated with the composition of the invention, will ingest the surfactant particles. Furthermore, the wetting of the compositions of the invention to a plant can be increased by the use of a wetting agent. Due to the stability of the surfactant particles against acids, bases and nucleases, the loaded surfactant particles of the invention provide resistance to the dsRNA against the digestive fluids in the digestive system of arthropods. Using the composition of the invention, dsRNA that is loaded in the surfactant particles can be transported through the digestive system of the insects. This makes the composition of the invention a suitable delivery system for nucleic acids (such as dsRNA) into arthropods.

In preferred embodiments, Compound A is a monomeric or a polymeric compound. The term polymer is meant to encompass oligomers.

In preferred embodiments, $R^1$ and $R^2$ are each independently selected from the group consisting of a $C_{1-6}$ alkyl, $C_{2-6}$ alkenyl, and $C_{2-6}$ alkynyl group; and/or $R^3$ and $R^4$ are each independently selected from the group consisting of a $C_{12-24}$ alkyl, a $C_{12-24}$ alkenyl, and a $C_{12-24}$ alkynyl group. Preferably, $R^1$ and $R^2$ are independently each selected from a $C_{1-6}$ alkyl group; and/or $R^3$ and $R^4$ are each independently selected from a $C_{12-24}$ alkyl group.

In preferred embodiments, $R^1$ and $R^2$ are each independently selected from a $C_{1-3}$ alkyl, $C_{2-3}$ alkenyl, and $C_{2-3}$ alkynyl group; and/or $R^3$ and $R^4$ are each independently selected from a $C_{12-22}$ alkyl, a $C_{12-22}$ alkenyl, and a $C_{12-22}$ alkynyl group.

In preferred embodiments, $R^1$ and $R^2$ are each independently selected from a $C_{1-3}$ alkyl group; and/or $R^3$ and $R^4$ each are independently selected from a $C_{12-22}$ alkyl group.

In preferred embodiments, $R^1$ and $R^2$ are methyl; and $R^3$ and $R^4$ are each independently selected from a $C_{12}$ alkyl and a $C_{18}$ alkyl group.

In preferred embodiments, Compound (A) is a didodecyldimethylammonium or dimethyldioctadecylammonium

cation. As mentioned elsewhere herein, the compositions of the invention are based on cationic ammonium derivatives (Compound A) that act as dsRNA complexation agents. These cationic ammonium-derivatives complex dsRNA that is negatively charged due to its phosphate groups by charge interaction. Additionally, Compound A used herein is a surfactant. Therefore, it is able to complex the dsRNA and forms protective surfactant particles that encompass the dsRNA.

In preferred embodiments, $R^5$, $R^6$, $R^7$ and $R^8$ are each independently selected from the group consisting of a linear or branched $C_{1-6}$ alkyl, $C_{2-6}$ alkenyl and $C_{2-6}$ alkynyl group; more preferably $C_{1-3}$ alkyl group, most preferably methyl.

In preferred embodiments, i and m are each independently selected from 1 to 12, more preferably wherein i is 4 to 8 and/or m is 2 to 4, most preferably wherein i is 6 and m is 3.

In preferred embodiments, n is 1 to 500, preferably 1 to 200, preferably 1 to 100, more preferably 1 to 50.

In preferred embodiments of the invention, Compound A comprises the structure

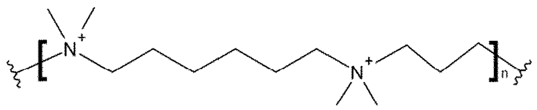

,

wherein n is 1 to 1000.

In preferred embodiments, the composition further comprises a stoichiometric amount of a negatively charged counter ion $X^-$ to the Compound A, preferably wherein the counter ion $X^-$ is a halogenide. If the plants are for human consumption, the counter ion is preferably non-toxic for human consumption. Preferably, the counter ion is chloride, bromide and/or iodide.

In preferred embodiments, Compound A is selected from the group consisting of 1,5-Dimethyl-1,5-diazaundecamethylene-polymethobromide, didodecyldimethylammonium bromide and dimethyldioctadecylammonium bromide.

In preferred embodiments of the invention, the surfactant particle additionally comprises

(i) Compound B, wherein Compound B is a non-ionic lipid; and/or

(ii) Compound C, wherein Compound C comprises an amphoteric phospholipid.

In particularly preferred embodiments, the surfactant particle additionally comprises Compound B and Compound C.

In the composition of the invention, Compound B functions as a dispersion stabilizer. The composition of the invention comprises surfactant particles. Preferably, it is a dispersion. Dispersion stabilizers are generally used in agrochemical formulations to ensure a sprayable homogenous dispersion of the active substances. Without wishing to be bound by theory, Compound B functions by being encompassed in the surfactant core (in the case of a micelle) or surfactant bilayer or multilayer membrane (in the case of a vesicle) of the surfactant particles of the invention. A surfactant particle that comprises a Compound B is called a lipid nanoparticle (LNP). The addition of Compound B provides beneficial effects to a composition of the invention. It increases the homogeneity of the surfactant particle mixture, prevents or reduces phase separation and agglomeration, and helps preserving the dispersion for extended periods of time. This is advantageous for storage of the composition of the invention, for example in a tank mixture.

In the composition of the invention, Compound C functions as a lipid bilayer or multilayer modifier. A lipid bilayer or multilayer modifier refers to a compound that influences the stability of a surfactant bilayer or multilayer, such as in the surfactant particles of the invention. Without wishing to be bound by theory, the amphoteric phospholipid functions by being encompassed in the surfactant core (in the case of a micelle) or surfactant bilayer or multilayer membrane (in the case of a vesicle) of the surfactant particles of the invention. A surfactant particle that comprises an amphoteric phospholipid lipid bilayer or multilayer modifier as used herein is called a lipid nanoparticle (LNP). Without wishing to be bound by theory, such a compound can modify the stability of an LNP by being encompassed in the lipid bilayer of multilayer.

In preferred embodiments, Compound B comprises the structure (B-I)

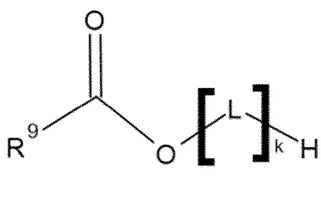

(B-I),

wherein

$R^9$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl, and $C_{2-24}$ alkynyl group is optionally substituted with one or more -OH;

$[L]_k$ is a polyoxyalkylene group comprising k repeating units, such as a polyoxymethylene group (referring to $[-(CH_2)-O-]_k$), a polyoxyethylene group (referring to $[-(C_2H_4)-O-]_k$), or a polyoxypropylene group (referring to $[-C_3H_6)-O-]_k$), or a copolymer of [L] comprising k oxyalkylene repeating units, preferably wherein the oxyalkylene repeating units are each independently selected from $[-(CH_2)-O-]$), $[-(C_2H_4)-O-]$ and $[-(C_3H_6)-O-]$, wherein k is 1 to 10000.

In preferred embodiments, k is 1 to 5000, preferably 1 to 2000, more preferably 1 to 1000, more preferably 1 to 500, even more preferably 1 to 100, more preferably 1 to 50, most preferably 1 to 20.

Preferably, $[L]_k$ is a polyoxyalkylene group comprising k repeating units selected from a polyoxyethylene group (referring to $[-(C_2H_4)-O-]_k$) and a polyoxypropylene group (referring to $[-C_3H_6)-O-]_k$), or a copolymer of [L] comprising k oxyalkylene repeating units, wherein the oxyalkylene repeating units are each independently selected from $[-(C_2H_4)-O-]$ and $[-(C_3H_6)-O-]$. Preferably, $[L]_k$ is $[-(C_2H_4)-O-]_k$, wherein k is 1 to 1000.

Preferably, the $R^9$ is selected from the group consisting of a $C_{12-20}$ alkyl, $C_{12-20}$ alkenyl and $C_{12-20}$ alkynyl group, wherein the $C_{12-20}$ alkyl, $C_{12-20}$ alkenyl and $C_{12-20}$ alkynyl group is optionally substituted with a -OH.

Preferably, the $R^9$ is a $C_{12-20}$ alkyl group, wherein the $C_{12-20}$ alkyl group is optionally substituted with a -OH.

Even more preferably, $R^9$ is a $C_{17}$ alkyl group, wherein the $C_{17}$ alkyl group is optionally substituted with a -OH. Most preferably, $R^9$ is $-(CH_2)_{10}-CH(OH)-(CH_2)_5-CH_3$.

In preferred embodiments, Compound B is a PEGylated fatty ester or PEGylated fatty alcohol. In preferred embodiments, the PEGylated fatty ester comprises the structure $R^{20}-COO[CH_2-CH_2-O]_p-H$, wherein $R^{20}$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl and $C_{2-24}$ alkynyl group is optionally substituted with one or more -OH, wherein p is 1 to 10000. In preferred embodiments, the PEGylated fatty alcohol comprises the structure $R^{20}-O[CH_2-CH_2-O]_p-H$, wherein $R^{20}$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl and $C_{2-24}$ alkynyl group is optionally substituted with one or more -OH, wherein p is 1 to 10000. Preferably, the $R^{20}$ is selected from the group consisting of a $C_{12-20}$ alkyl, $C_{12-20}$ alkenyl and $C_{12-20}$ alkynyl group, wherein the $C_{12-20}$ alkyl, $C_{12-20}$ alkenyl and $C_{12-20}$ alkynyl group is optionally substituted with a -OH. Preferably, the $R^{20}$ is a $C_{12-20}$ alkyl group, wherein the $C_{12-20}$ alkyl group is optionally substituted with a -OH. Even more preferably, $R^{20}$ is a $C_{17}$ alkyl group, wherein the $C_{17}$ alkyl group is optionally substituted with a -OH. Most preferably, $R^{20}$ is $-(CH_2)_{10}-CH(OH)-(CH_2)_5-CH_3$. In preferred embodiments, p is 1 to 5000, preferably 1 to 2000, more preferably 1 to 1000, more preferably 1 to 500, even more preferably 1 to 100, more preferably 1 to 50, most preferably 1 to 20.

In preferred embodiments of the invention, the composition, preferably the surfactant particle, additionally comprises a wetting agent D, wherein the wetting agent D is selected from the group consisting of a non-ionic surfactant, a cationizable surfactant and a cationic surfactant.

In the composition of the invention, the wetting agent D functions by reducing the interfacial tension between a liquid and a surface and therefore increases spreading of the liquid on the surface. Wetting agents are used in plant protection to provide spreading, i.e. complete coverage of the applied active ingredients on the plant. The addition of wetting agents to the compositions of the invention facilitates uptake by feeding insects.

The wetting agent D of the invention is selected from the group consisting of a non-ionic surfactant, a cationizable surfactant and a cationic surfactant. In the context of the invention, the "cationizable" surfactant refers to a molecule that is in an equilibrium between a molecular state with a free electron pair and a protonated cationic molecular state, wherein the molecule is mainly in the protonated cationic molecular state under acidic conditions. Preferably, the cationizable surfactant is an amine derivative.

In preferred embodiments of the invention, the wetting agent D is a non-ionic surfactant. In preferred embodiments, non-ionic surfactant is selected from the group consisting of an ethoxylated and/or propoxylated alcohol; and a polyether modified trisiloxane.

In preferred embodiments, the cationizable surfactant is a tertiary amine-derivative. In preferred embodiments of the invention, the cationic surfactant is a quaternary ammonium-derivative.

In preferred embodiments, the tertiary amine-derivative comprises the structure (D-IIIb) and/or the quaternary ammonium-derivative comprises the structure (D-IIIa)

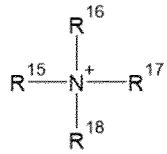 (D-IIIa), 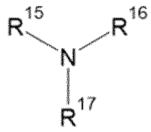 (D-IIIb),

wherein

$R^{18}$ is selected from the group consisting of a linear or branched $C_{1-24}$ alkyl, $C_{2-24}$ alkenyl and $C_{2-24}$ alkynyl group, and

$R^{15}$, $R^{16}$ and $R^{17}$ are each independently selected from the group consisting of a linear or branched $C_{1-24}$ alkyl, $C_{2-24}$ alkenyl and $C_{2-24}$ alkynyl group.

In preferred embodiments, the tertiary amine-derivative comprises the structure (D-IIIb) and/or the quaternary ammonium-derivative comprises the structure (D-IIIa)

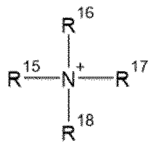 (D-IIIa) 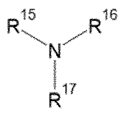 (D-IIIb),

wherein

$R^{18}$ is selected from the group consisting of a linear or branched $C_{1-3}$ alkyl group, and

$R^{15}$ and $R^{16}$ are each independently selected from a $C_{1-3}$ alkyl group and $R^{17}$ is selected from a $C_{10-20}$ alkyl, $C_{10-20}$ alkenyl and $C_{10-20}$ alkynyl group.

Preferably, the tertiary amine-derivative comprises the structure (D-IIIb) and/or the quaternary ammonium-derivative comprises the structure (D-IIIa)

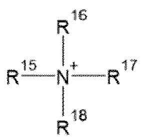 (D-IIIa) 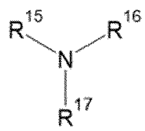 (D-IIIb),

wherein

$R^{18}$ is selected from the group consisting of a linear or branched $C_{1-3}$ alkyl group, and

$R^{15}$ and $R^{16}$ are each independently selected from a $C_{1-3}$ alkyl group and $R^{17}$ is selected from a $C_{14-20}$ alkyl group.

In preferred embodiments, the wetting agent D is N,N'-Dimethyltetradecylamine (DMTDA) and/or Cetyltrimethylammonium bromide (CTAB).

In preferred embodiments, the ethoxylated and/or propoxylated alcohol comprises the structure (D-I)

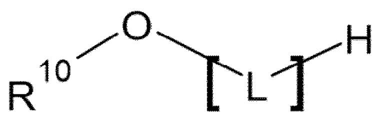

(D-I),

wherein $R^{10}$ is selected from the group consisting of a linear or branched $C_{8-24}$ alkyl, $C_{8-24}$ alkenyl and $C_{8-24}$ alkynyl group,

[L] is a polyoxyalkylene group comprising k repeating units, such as a polyoxymethylene group (referring to $[-(CH_2)-O-]_b$), a polyoxyethylene group (referring to $[-(C_2H_4)-O-]_b$), or a polyoxypropylene group (referring to $[-(C_3H_6)-O-]_b$), or a copolymer comprising b oxyalkylene repeating units, preferably wherein the oxyalkylene repeating units are each independently selected from $[-(CH_2)-O-]$), $[-(C_2H_4)-O-]$ and $[-(C_3H_6)-O-]$;

and b is 1 to 10000.

In preferred embodiments, the ethoxylated and/or propoxylated alcohol comprises the structure (D-I)

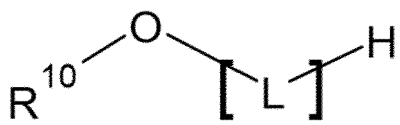

(D-I),

wherein $R^{10}$ is selected from the group consisting of a linear or branched linear or branched $C_{8-12}$ alkyl, $C_{8-12}$ alkenyl and $C_{8-12}$ alkynyl group,

[L] is a polyoxyethylene group selected from $[-(C_2H_4)-O-]_b$, a polyoxypropylene group selected from $[-(C_3H_6)-O-]_b$ or a copolymer comprising b oxyalkylene repeating units that are each independently selected from $[-(C_2H_4)-O-]$ and $[-(C_3H_6)-O-]$,

and b is 1 to 10000.

In preferred embodiments, b is 1 to 5000, preferably 1 to 2000, more preferably 1 to 1000, more preferably 1 to 500, even more preferably 1 to 100, more preferably 1 to 50, most preferably 1 to 20.

Preferably, $R^{10}$ is a $C_{8-12}$ alkyl group, preferably $R^{10}$ is a $C_8$ alkyl group, more preferably a branched $C_8$ alkyl group.

In preferred embodiments of the invention, the wetting agent is

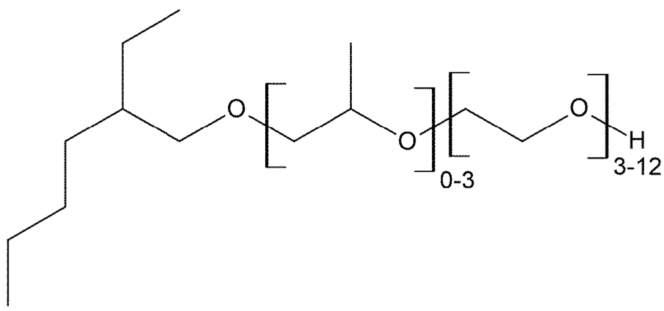

and comprises a random or block-like distribution of the repeating units

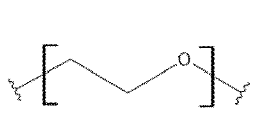

and

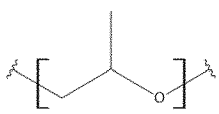

.

In preferred embodiments, the polyether-modified trisiloxane comprises the structure (D-II):

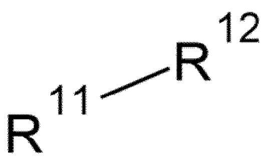

(D-II),

wherein

$R^{11}$ is a linear trisiloxane with the structure $R^{11'}_3$Si-O-Si$R^{11'}_2$-O-Si$R^{11'}_3$, or a cyclic trisiloxane with the structure [O-Si$R^{11'}_2$]$_3$, wherein each of the $R^{11'}$ is independently selected form the group consisting of $C_{1-24}$ alkyl, $C_{2-24}$ alkenyl and $C_{2-24}$ alkynyl group,

$R^{12}$ is a substituent bound to $R^{11'}$ and is a polyether, such as a polyethylene glycol or a polypropylene glycol or a copolymer thereof, with 1 to 10.000 repeating units, optionally wherein the polyether is terminated with a methyl group.

In preferred embodiments, each of the $R^{11'}$ is independently selected form the group consisting of $C_{1-12}$ alkyl, $C_{2-12}$ alkenyl and $C_{2-12}$ alkynyl group; more preferably, wherein each $R^{11'}$ is independently selected form the group consisting of $C_{1-6}$ alkyl, $C_{2-6}$ alkenyl and $C_{2-6}$ alkynyl group.

In preferred embodiments, each of the $R^{11'}$ is independently selected form the group consisting of $C_{1-12}$ alkyl, $C_{2-12}$ alkenyl and $C_{2-12}$ alkynyl group; more preferably, wherein each $R^{11'}$ is independently selected from the group consisting of a $C_{1-6}$ alkyl group. Most one or more of the $R^{11'}$ is a methyl. In the case that $R^{11'}$ is a methyl group, $R^{12}$ is optionally a substituent of said methyl group.

In preferred embodiments, $R^{12}$ is a polyether with 1 to 500 repeating units, preferably with 1 to 200 repeating units, more preferably with 1 to 100 repeating units, more preferably with 1 to 50 repeating units, most preferably with 1 to 20 repeating units, optionally wherein the polyether is terminated with a methyl group.

In preferred embodiments, the $R^{11}$ is a linear trisiloxane and $R^{11'}$ is a $C_{1-3}$ alkyl group.

The compositions of the invention are compatible with wetting agents including amine and ammonium-derived surfactants and wetting agents that are based on ethoxylated and/or propoxylated alcohols and siloxanes such as trisiloxanes. On the other hand, the LNPs of the invention are not compatible with anionic surfactants such as sodium dodecyl sulfate. Without wishing to be bound by theory, such anionic wetting agents interact with the positively charged Compound A, which leads to a decomplexation of the dsRNA.

Compound C comprises an amphoteric phospholipid. In particular preferred embodiments, Compound C is an amphoteric phospholipid.

By using an amphoteric phospholipid, it is possible to influence the stability of the surfactant particles of the invention. For example, phosphatidylcholine is known to increase the stability of LNP membranes, whereas phosphatidylethanolamine is known to increase membrane-curvature and to increase tension a LNP membrane, increasing its fusion capability.

In preferred embodiments the amphoteric phospholipid comprises a compound selected from the group consisting of a phosphatidylcholine, a phosphatidylethanolamine, lysophosphatidylcholine, and a sphingomyelin.

Preferably, the phosphatidylcholine comprises the formula (C-I):

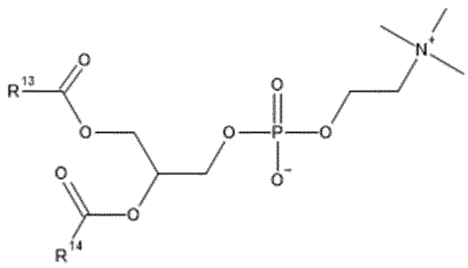

(C-I),

wherein $R^{13}$ and $R^{14}$ are each independently selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group.

Preferably, the phosphatidylethanolamine comprises the formula (C-II)

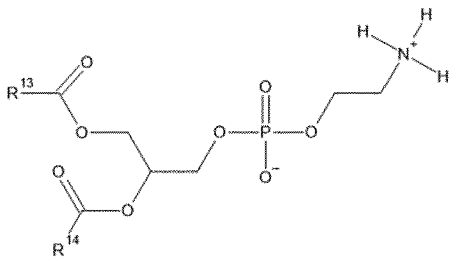

(C-II);

wherein $R^{13}$ and $R^{14}$ are independently each selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group.

In preferred embodiments, the Compound C is egg-lecithin. Preferably the egg-lecithin comprises with relation to the overall mass of the egg-lecithin one or more, preferably all, of the following components:

Phosphatidylcholine with a mass-fraction of 50 - 85 %;

Phosphatidylethanolamine with a mass-fraction of 10.0 - 20.0 %;

Lysophosphatidylcholine with a mass-fraction of ≤4 %;

Sphingomyelin with a mass-fraction of ≤4 %.

More preferably the egg-lecithin comprises with relation to the overall mass of the egg-lecithin one or more, preferably all, of the following mass-based components:

Phosphatidylcholine with a mass-fraction of 64.0 - 79.0 %;

Phosphatidylethanolamine with a mass-fraction of 12.0 - 18.0 %;

Lysophosphatidylcholine with a mass-fraction of ≤3 %;

Sphingomyelin with a mass-fraction of ≤3 %.

In preferred embodiments of the invention, the surfactant particle additionally comprises a further lipid bilayer or multilayer stabilizer E.

In preferred embodiments, the surfactant particle of the invention comprises a further lipid bilayer or multilayer stabilizer modifier E, wherein the further lipid bilayer or multilayer modifier is selected from the group consisting of a cholesterol and derivatives thereof. Preferably, this additional lipid bilayer or multilayer stabilizer is cholesterol. Said lipid bilayer or multilayer stabilizer as indicated herein functions by increasing the stability of the lipid bilayer or multilayer that comprises the lipid bilayer or multilayer stabilizer, for examples as found in liposomes. By the inclusion of cholesterol in a lipid bilayer or multilayer, the stability of the lipid bi- or multilayer is increased. Without wishing to be bound by theory, the cholesterol is integrated within the lipid bilayer of such an LNP where its rigid steroid rings interact with the regions of the hydrocarbon chains in the vicinity the polar surfactant headgroups. This results in the lipid bilayer being less deformable in this region and thereby decreasing the permeability of the bilayer to small water-soluble molecules. Furthermore, it can inhibit phase transitions of the lipid-bilayer increasing the overall stability of the LNPs.

In the context herein, the "N/P ratio" refers to the molar amount of nitrogen atoms from Compound A in the composition to the molar amount of phosphorous atoms from the dsRNA. N is the molar amount of nitrogen atoms from Compound A in the composition. P is the molar amount of phosphorus atoms from the dsRNA in the composition. Therefore, the N/P ratio is calculated as follows:

$$N/P = \frac{\text{molar amount of nitrogen atoms from Compound A}}{\text{molar amount of phosphorus atoms from the dsRNA}}$$

In preferred embodiments, the composition comprises a N/P ratio of N/P > 1; preferably of N/P = 1 to 5, more preferably of N/P = 1 to 3.5.

In preferred embodiments, the composition comprises a detection agent, preferably wherein the detection agent is a molecule or moiety that can be detected via a UV, fluorescence, luminescence and/or radioactive, mass signal. In particular preferred embodiments, the detection label can be detected via mass spectrometry. For example, the detection agent may be an isotopically-labelled compound or a chelator for lanthanoids. Such embodiments are useful to track the biodistribution of the composition of the invention in the arthropod. In preferred embodiments, the detection agent is covalently bound to the dsRNA. These embodiments are useful to track the biodistribution of the dsRNA in the arthropod.

In preferred embodiments, the Compound A, Compound B and Compound C have a molar ratio of A:B:C of (35-65):(15-35):(15-35), preferably (40-60):(20-30):(20-30), more preferably (50-60):(20-30):(20-30). The inventors have found that compositions according to the invention with these molar ratios of A:B:C perform well in forming emulsions that

are stable against various surfactants.

In preferred embodiments the Compound A, Compound B and Compound C have a molar ratio of A:B:C of (40-60):(20-30):(20-30), preferably (45-55):(20-30):(20-30), preferably (48-52):(23-27):(23-27). The inventors demonstrate that compositions according to the invention with these molar ratios of A:B:C feature stability and uptake of mosquito larvae. Preferably, the composition of the invention is applied to the leaves of a plant. This application is particularly advantageous, as the leaves feature a high surface area. The inventors also demonstrate herein that these compositions are stable against pH values and a model nuclease, that mimic the conditions in the digestive system of arthropods, e.g. insects. After ingestion by such arthropods, said surfactant particles of the invention protect the dsRNA against the digestive system of the arthropods and are up taken by these animals. Moreover, the dsRNA particles accumulate in the head region of filter insects, such as of mosquito larvae, after ingestion of the said surfactant particles.

In particular preferred embodiments of the invention, the surfactant particles of the composition that encompass the dsRNA for gene silencing against an arthropod target gene comprise Compound A, Compound B, Compound C.

In even more particular preferred embodiments of the invention, the surfactant particles of the composition that encompass the dsRNA for gene silencing against an arthropod target gene comprise Compound A, Compound B, Compound C and the wetting agent D.

and the wetting agent Compound D as defined herein.

In preferred embodiments, the dsRNA is a sequence that comprises 20 to 2000 base pairs, preferably wherein the dsRNA is a sequence that comprises 100 to 1000 base pairs, more preferably the dsRNA is a sequence that comprises 400 to 500 base pairs.

In preferred embodiments of the invention, the composition additionally comprises a mineral agent F and/or a metal-ion chelating agent.

In preferred embodiments, the mineral agent comprises a cation selected from the group consisting of $Li^+$, $Na^+$, $K^+$, $Mg^{2+}$, $Ca^{2+}$, $Fe^{2+}$, $Fe^{3+}$, $Zn^{2+}$, $Ni^+$, $Ni^{2+}$, $Cu^+$, and $Cu^{2+}$ and/or comprises an anion selected from the group consisting of $F^-$, $Cl^-$, Br, $I^-$, $PO_4^{3-}$, $HPO_4^{2-}$, $H_2PO_4^-$, $SO_3^{2-}$, $SO_4^{2-}$, formiate, acetate and propionate anions. Such mineral agents can be present in the composition of the invention, depending on the buffer used in the composition. They can be added to stabilize the dsRNA.

In the context of the invention, the metal-ion chelating agent is a polydentate ligand capable of forming two or more separate coordination bonds to a single central atom. In the composition of the invention, the metal-ion chelating agent functions by sequestering divalent metal-ions that are necessary for enzymatic nuclease activity. Therefore, adding a metal-ion chelating agent inhibits enzymatic nuclease activity and further protects the dsRNA from degradation.

In preferred embodiments, the composition further includes one or more of

(i) a thickener;

(ii) a formulation adhesive; and/or

(iii) a freeze-drying stabilizer.

The term "thickener" as used herein refers to a rheological flow modifier that increases the viscosity of the composition of the invention. Preferably, the thickener is a gelling agent. A gelling agent is a compound that may be added to a formulation or a concentrate thereof to increase the viscosity of the formulation or the concentrate and to prevent phase separation of the formulation components. Preferably, the thickener is a polysaccharide or a natural polypeptide such as a silk protein. Preferably the thickener is a mineral, preferably an insoluble mineral such as a clay or silica.

The term "formulation adhesive" as used herein refers to a component that reduces the washing of the formulation from the plant, for example by rain. Preferably, the formulation adhesive is selected from a polysaccharide, a polypeptide, a polyisoprene, and a polybutylene.

The term "freeze-drying stabilizer" refers to a compound that is added to the composition of the invention to prevent damage to the formulation components and particulate structure during freeze-drying procedure. This is advantageous to produce re-dispersible powder formulations. Preferably, the freeze-drying stabilizer comprises a soluble polymer and/or a small molecular compound, preferably wherein the freeze-drying stabilizer is a polyglycol (such as polyethylene glycol), a saccharide such as glucose or a polysaccharide such as dextran.

Preferably, the composition of the invention is in a form selected from the group consisting of a solid, a liquid, a powder, a suspension, an emulsion, a spray, a carrier particulate, a film, a matrix, a soil drench, an insect diet or insect bait, and seed treatment. In particular, the composition of the invention is in the form of an aqueous suspension or emulsion. In preferred embodiments of the invention, the composition of the invention is formulated for spray application, foliar application, granular application, pouring application, drip application, broadcast application, aerial application, electrostatic application or injection application. Most preferably, the composition is formulated for spray application.

In preferred embodiments of the invention, the composition of the invention is a specific pest control composition

against a target arthropod. In other words, the composition of the invention is a pest control composition that affects the target arthropod but does affect to a lesser degree or does not affect a non-target arthropod. Preferably, the arthropod target gene is species-specific for the target arthropod.

In preferred embodiments, the arthropod is an insect. Preferably, the arthropod is in a larvae state or adult state. More preferably, the arthropod is in a larvae state.

In preferred embodiments, wherein the arthropod is an organism of the order *Diptera, Hemiptera, Blattodea, Orthoptera, Coleoptera, Thysanoptera, Hymenoptera, Lepidoptera and/or Phthiraptera.* More preferably, the arthropod is a pest insect. In preferred embodiments, the arthropod belongs to the order of *Diptera,* more preferably to the family of *Culicidae,* wherein the arthropod belongs to the genus of *Aedes,* most preferably wherein the arthropod belongs to species of *Aedes albopictus.*

In preferred embodiments, the arthropod is a vector arthropod, for example a mosquito.

**In a second aspect,** the invention pertains to a use of a composition for the delivery of a dsRNA to an arthropod, wherein the composition comprises a surfactant particle with an average hydrodynamic diameter of 20 to 200 nm when determined by dynamic light scattering, wherein the surfactant particle encompasses a double-stranded RNA (dsRNA), wherein the surfactant particle comprises

Compound A with the structure (A-I) or (A-II);

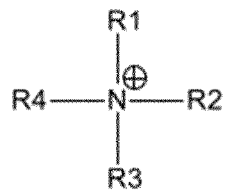

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group;

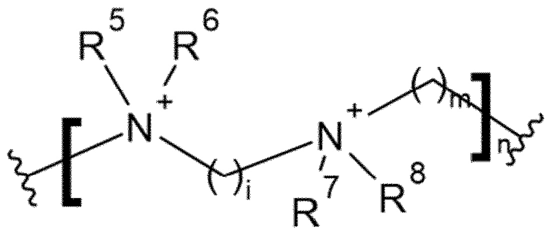

(A-II),

wherein

$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;

i and m are each independently selected from 1 to 24; and

n is 1 to 1000, and

wherein

the use comprises a step of oral ingestion of the composition by the arthropod.

Preferably, Compound A is as defined according to the first aspect of the invention.

In preferred embodiments of the use of the second aspect, the dsRNA is for gene silencing against an arthropod target gene, preferably wherein gene silencing is RNAi. In these embodiments, the composition is a plant protection agent against the arthropod.

Preferably, the arthropod target gene is as defined according to the first aspect of the invention. In preferred embodiments, the composition used in the second aspect comprises any feature of the composition of the first aspect of the invention. In preferred embodiments, the surfactant particle is a surfactant particle as defined in the first aspect of the invention. In preferred embodiments of the invention, the arthropod is an arthropod as defined according to the first aspect of the invention. Preferably, the arthropod is a pest insect. Preferably, the arthropod is a vector insect.

Preferably, the surfactant particle in the composition used in the second aspect of the invention additionally comprises

(i) Compound B, wherein Compound B is a non-ionic lipid according ot the first aspect of the invention; and/or

(ii) Compound C, wherein Compound C comprises an amphoteric phospholipid according to the first aspect of the invention;

preferably wherein surfactant particle in the composition used in the second aspect of the invention additionally comprises Compound B and Compound C.

Preferably, the composition used in the second aspect of the invention, preferably the surfactant particle, additionally comprises a wetting agent D, wherein the wetting agent D is selected from the group consisting of a non-ionic surfactant, a cationizable surfactant and a cationic surfactant as defined according to the first aspect of the invention.

Preferably, the surfactant particle in the composition used in the second aspect of the invention additionally comprises a further lipid bilayer or multilayer stabilizer E as defined according to the first aspect of the invention.

Preferably, the composition used in the second aspect of the invention additionally comprises a mineral agent F and/or a metal-ion chelating agent as defined according to the first aspect of the invention.

Preferably, the composition used in the second aspect of the invention additionally comprises one or more of

(i) a thickener

(ii) a formulation adhesive; and/or

(iii) a freeze-drying stabilizer.

In preferred embodiments, the composition of the second aspect of the invention is a composition according to the first aspect of the invention.

In preferred embodiments of the invention, the use comprises a step of applying the composition of the invention to a plant. Preferably the step of applying the composition of the invention to a plant comprises one or more of spray application, foliar application, granular application, pouring application, drip application, broadcast application, aerial application, electrostatic application or injection application. Most preferably, the composition is applied to the plant by spray application.

The term "plant" as used herein includes whole plants, ancestors and progeny of the plants and plant parts, including harvestable parts of the plant. Preferably, the plant part is selected from the group consisting of seeds, shoots, stems, leaves, roots (including tubers), flowers, and tissues and organs. Preferably, the plant is a plant that belongs to the superfamily Viridiplantae.

The methods of the invention are advantageously applicable to any plant, in particular to any plant as defined herein. Plants that are particularly useful in the methods of the invention include all plants which belong to the superfamily Viridiplantae, preferably to order of Caryophyllales. Most preferably, the plant is a sugar beet.

Preferably, the composition of the invention is applied to a plant tissue, plant organ, plant epidermis, or any other part of a plant. The plant organ may be a leaf, stem, root, or reproductive organ. The plant tissue can be any tissue, including meristematic tissue. Most preferably, the composition of the invention is applied to a leaf of the plant. Generally, the leaves are a high surface area part of a plant, therefore a large amount of the composition of the invention can be applied to said part.

In preferred embodiments, the use comprises a step of oral ingestion of the composition by the arthropod. In preferred embodiments of the invention, the surfactant particle of the composition is transported through the arthropod digestive system, preferably through the insect digestive system. The composition of the invention is particularly useful for such a route of uptake. As mentioned elsewhere herein, the composition of the invention comprises surfactant particles that provide protection to the dsRNA encompassed by said surfactant particles. In preferred embodiments of the invention,

the composition of the invention protects the dsRNA therein against the environment after being spray applied to plants, in particular against nuclease that are present in the environment and/or the digestive system of arthropods.

In preferred embodiments of the invention, the use comprises a step of spraying the of the composition onto a plant and a subsequent step of oral ingestion of the composition by the arthropod.

Preferably, the dsRNA is delivered to the head region of the larvae of *Aedes albopictus,* such as when applied in water to swimming mosquito larvae.

In preferred embodiments, the composition is a composition according to the first aspect of the invention.

**In a third aspect, the invention pertains to a method of manufacturing** a composition of the first aspect of the invention, wherein the method comprises mixing a solution 1 comprising a Compound A, a Compound B and a Compound C with a solution 2 comprising the dsRNA.

In preferred embodiments of the method of the third aspect, the solution 1 comprises an organic solvent such as ethanol and/or the solution 2 is an aqueous solution.

In preferred embodiments of the third aspect, the mixing is conducted in a microfluidic reactor.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of", both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by $\pm 20\%$, $\pm 15\%$, $\pm 10\%$, and for example $\pm 5\%$. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

All references, patents, and publications cited herein are hereby incorporated by reference in their entirety.

In view of the above, it will be appreciated that the present invention also relates to the following itemised embodiments:

Item 1. A composition, comprising a surfactant particle with an average hydrodynamic diameter of 20 to 200 nm as determined by dynamic light scattering, wherein the surfactant particle encompasses a double-stranded RNA (dsRNA) for gene silencing against an arthropod target gene, wherein the surfactant particle comprises Compound A with the structure (A-I) or (A-II);

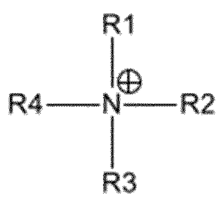

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group;
or

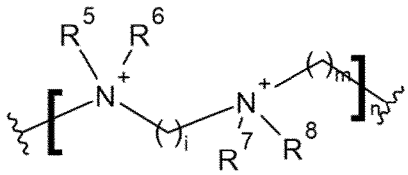

(A-II),

wherein
$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;
i and m are each independently selected from 1 to 24; and
n is 1 to 1000.

Item 2: The composition of item 1, wherein the surfactant particle additionally comprises

(i) Compound B, wherein Compound B is a non-ionic lipid; and/or
(ii) Compound C, wherein Compound C comprises an amphoteric phospholipid;

preferably wherein the surfactant particle comprises Compound B and Compound C.

Item 3: The composition of item 1 or 2, wherein the composition, preferably the surfactant particle, additionally comprises a wetting agent D, wherein the wetting agent D is selected from the group consisting of a non-ionic surfactant, a cationizable surfactant and a cationic surfactant.

Item 4: The composition of any one of items 1 to 3, wherein the surfactant particle additionally comprises a lipid bilayer or multilayer stabilizer E selected from the group consisting of a cholesterol and derivatives thereof; and/or

Item 5: The composition of any one of items 1 to 4, wherein the composition additionally comprises an additional agent F selected from the group consisting of a mineral agent F and/or a metal ion chelating agent.

Item 6: The composition of any one of items 1 to 5, wherein

$R^1$ and $R^2$ are each independently selected from the group consisting of a $C_{1-6}$ alkyl, $C_{2-6}$ alkenyl, and $C_{2-6}$ alkynyl group; and/or

$R^3$ and $R^4$ are each independently selected from the group consisting of a $C_{12-24}$ alkyl, a $C_{12-24}$ alkenyl, and a $C_{12-24}$ alkynyl group.

Item 7: The composition of any one of items 1 to 6, wherein

$R^1$ and $R^2$ are each independently selected from the group consisting of a $C_{1-3}$ alkyl, $C_{2-3}$ alkenyl, and $C_{2-3}$ alkynyl group; and/or

$R^3$ and $R^4$ are each independently selected from the group consisting of a $C_{12-22}$ alkyl, a $C_{12-22}$ alkenyl, and a $C_{12-22}$ alkynyl group.

Item 8: The composition of any one of items 1 to 7, wherein

$R^1$ and $R^2$ are methyl; and

$R^3$ and $R^4$ are each independently selected from a $C_{12}$ alkyl and a $C_{18}$ alkyl group.

Item 9: The composition of any one of items 1 to 8, wherein Compound A is a didodecyldimethylammonium or

dimethyldioctadecylammonium cation.

Item 10: The composition of items 1 to 9, wherein $R^5$, $R^6$, $R^7$ and $R^8$ are each independently selected from the group consisting of a linear or branched $C_{1-6}$ alkyl, $C_{2-6}$ alkenyl and $C_{2-6}$ alkynyl group; more preferably $C_{1-3}$ alkyl group, most preferably a methyl group.

Item 11: The composition of items 1 to 8, wherein i and m are each independently selected from 1 to 12, more preferably wherein i is 4 to 8 and/or m is 2 to 4, most preferably wherein i is 6 and m is 3.

Item 12: The composition of item 1 to 9, wherein n is 1 to 500, preferably 1 to 200, preferably 1 to 100, more preferably 1 to 50.

Item 13: The composition of any one of claims 1 to 10, wherein Compound A comprises the structure

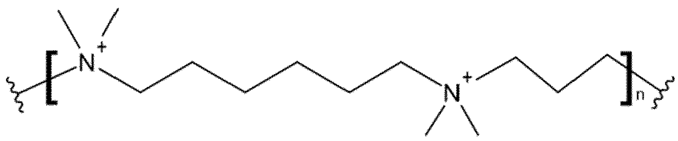

,

wherein n is 1 to 1000.

Item 14: The composition of claim 1 to 13, further comprising a stoichiometric amount of a negatively charged counter ion $X^-$ to the Compound A, preferably wherein the counter ion $X^-$ is a halogenide, more preferably, wherein the counter ion is a chloride, bromide and/or iodide.

Item 15: The composition of claim 1 to 14, wherein Compound A is selected from the group consisting of 1,5-Dimethyl-1,5-diazaundecamethylene-polymethobromide, didodecyldimethylammonium bromide and dimethyldioctadecylammonium bromide.

Item 16: The composition of any one of claims 2 to 15, wherein Compound B comprises the structure (B-I)

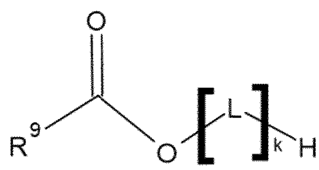

(B-I),

wherein

$R^9$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl and $C_{2-24}$ alkynyl group is optionally substituted with one or more -OH;

$[L]_k$ is a polyoxyalkylene group comprising k repeating units, such as a polyoxymethylene group (referring to $[-(CH_2)-O-]_k$), a polyoxyethylene group (referring to $[-(C_2H_4)-O-]_k$), or a polyoxypropylene group (referring to $[-C_3H_6)-O-]_k$), or a copolymer of [L] comprising k oxyalkylene repeating units, preferably wherein the oxyalkylene repeating units are each independently selected from the group consisting of $[-(CH_2)-O-])$, $[-(C_2H_4)-O-]$ and $[-(C_3H_6)-O-]$;

wherein k is 1 to 10000.

Item 17: The composition of any one of claims 2 to 16, wherein Compound B is a PEGylated fatty alcohol.

Item 18: The composition of claim 17, wherein the PEGylated fatty alcohol comprises the structure $R^{20}-O[CH_2-C]_p-H$, wherein $R^{20}$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl and $C_{2-24}$ alkynyl group is optionally substituted with one or more -OH, wherein p is 1 to 10000.

Item 19: The composition of any one of claims 2 to 18, wherein the amphoteric phospholipid comprises a compound selected from of the group consisting of a phosphatidylcholine, a phosphatidylethanolamine, lysophosphatidylcholine, and a sphingomyelin.

Item 20: The composition of claim 19, wherein the phosphatidylcholine comprises the formula (C-I),

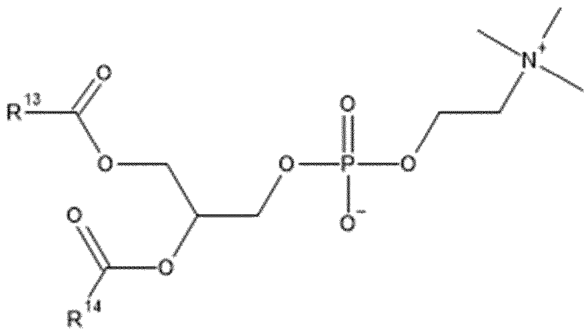

(C-I),

wherein $R^{13}$ and $R^{14}$ are each independently selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group.

Item 21: The composition of item 19 or 20, wherein the phosphatidylethanolamine comprises the formula (C-II),

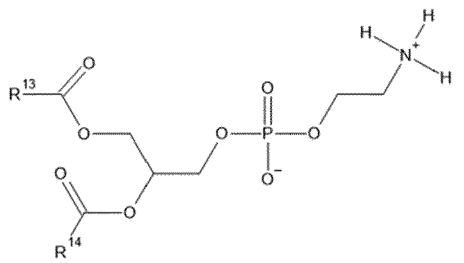

(C-II),

wherein $R^{13}$ and $R^{14}$ are each independently selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group.

Item 22: The composition of any one of items 2 to 21, wherein the Compound C is egg-lecithin.

Item 23: The composition of any one of items 3 to 22, wherein the wetting agent D is the non-ionic surfactant.

Item 24: The composition of any one of items 3 to 23, wherein the non-ionic surfactant is selected from the group consisting of an ethoxylated and/or propoxylated alcohol; and a polyether modified trisiloxane.

Item 25: The composition of item 24, wherein the ethoxylated and/or propoxylated alcohol comprises the structure (D-I)

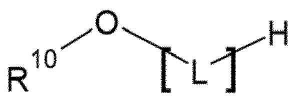

(D-I),

wherein $R^{10}$ is a linear or branched linear or branched $C_{8-24}$ alkyl, $C_{8-24}$ alkenyl and $C_{8-24}$ alkynyl group,

[L] is a polyoxyalkylene group comprising k repeating units, such as a polyoxymethylene group (referring to $[-(CH_2)-O-]_b$), a polyoxyethylene group (referring to $[-(C_2H_4)-O-]_b$), or a polyoxypropylene group (referring to $[-C_3H_6)-O-]_b$), or a copolymer comprising k oxyalkylene repeating units, preferably wherein the oxyalkylene

repeating units are independently selected from the group consisting of [-($CH_2$)-O-], [-($C_2H_4$)-O-] and [-($C_3H_6$)-O-];

and b is 1 to 10000.

Item 26: The composition of any one of items 24 or 25, wherein the ethoxylated and/or propoxylated alcohol comprises the structure (D-I)

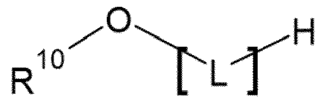

(D-I),

wherein $R^{10}$ is selected from the group consisting of a linear or branched linear or branched $C_{8-12}$ alkyl, $C_{8-12}$ alkenyl and $C_{8-12}$ alkynyl group,

[L] is a polyoxyethylene group such as [-($C_2H_4$)-O-]$_b$, a polyoxypropylene group such as [-($C_3H_6$)-O-]$_b$ or a copolymer comprising b oxyalkylene repeating units that are each independently selected from the group consisting of [-($C_2H_4$)-O-] and [-($C_3H_6$)-O-],

and b is 1 to 10000.

Item 27: The composition of item 25 or 26, wherein b is 1 to 5000, preferably 1 to 2000, more preferably 1 to 1000, more preferably 1 to 500, even more preferably 1 to 100, more preferably 1 to 50, most preferably 1 to 20.

Item 28: The composition of any one of items 24 to 27, wherein the polyether-modified trisiloxane comprises the structure (D-II):

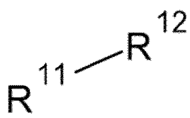

(D-II),

wherein

$R^{11}$ is a linear trisiloxane with the structure $R^{11'}_3$Si-O-Si$R^{11'}_2$-O-Si$R^{11'}_3$, or a cyclic trisiloxane with the structure [O-Si$R^{11'}_2$]$_3$, wherein each of the $R^{11'}$ is independently selected from the group consisting of $C_{1-24}$ alkyl, $C_{2-24}$ alkenyl and $C_{2-24}$ alkynyl group,

$R^{12}$ is a substituent bound to $R^{11'}$ and is a polyether, such as a polyethylene glycol or a polypropylene glycol or a copolymer thereof, with 1 to 10.000 repeating units, optionally wherein the polyether is terminated with a methyl group.

Item 29: The composition of item 28, wherein each of the $R^{11'}$ is independently selected from the group consisting of $C_{1-12}$ alkyl, $C_{2-12}$ alkenyl or $C_{2-12}$ alkynyl group; more preferably, wherein each $R^{11'}$ is independently selected form the group consisting of $C_{1-6}$ alkyl group; most preferably wherein each of the $R^{11'}$ is a methyl group.

Item 30: The composition of item 28 or 29, wherein $R^{12}$ is a polyether with 1 to 500 repeating units, preferably with 1 to 200 repeating units, more preferably with 1 to 100 repeating units, more preferably with 1 to 50 repeating units, most preferably with 1 to 20 repeating units, optionally wherein the polyether is terminated with a methyl group.

Item 31: The composition of any one of items 28 to 30, wherein $R^{11}$ is a linear trisiloxane and $R^{11'}$ is a $C_{1-3}$ alkyl group.

Item 32: The composition of any one of items 3 to 22, wherein the wetting agent D is the cationizable surfactant and/or the cationic surfactant.

Item 33: The composition of any one of items 3 to 32, wherein the cationizable surfactant is a tertiary amine-derivative and/or, wherein the cationic surfactant is a quaternary ammonium-derivative.

Item 34: The composition of item 33, wherein the tertiary amine-derivative comprises the structure(D-IIIb) and/or the quaternary ammonium-derivative comprises the structure (D-IIIa)

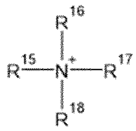 (D-IIIa), 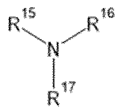 (D-IIIb)

wherein

$R^{18}$ is selected from the group consisting of a linear or branched $C_{1-24}$ alkyl, $C_{2-24}$ alkenyl and $C_{2-24}$ alkynyl group, and

$R^{15}$, $R^{16}$ and $R^{17}$ are each independently selected from the group consisting of a linear or branched $C_{1-24}$ alkyl, $C_{2-24}$ alkenyl and $C_{2-24}$ alkynyl group.

Item 35: The composition of any one of item 34, wherein

$R^{18}$ is selected from the group consisting of a linear or branched $C_{1-3}$ alkyl group, and

$R^{15}$ and $R^{16}$ are each independently selected from the group consisting of $C_{1-3}$ alkyl group and $R^{17}$ is selected from the group consisting of $C_{10-20}$ alkyl, $C_{10-20}$ alkenyl and $C_{10-20}$alkynyl group.

Item 36: The composition of any one of items 4 to 35, wherein the further lipid bilayer or multilayer stabilizer E is cholesterol and/or a cholesterol derivative.

Item 37: The composition of any one of items 1 to 36, wherein the surfactant particle is a lipid nanoparticle (LNP), preferably a liposome.

Item 38: The composition of any one of items 1 or 37, wherein the surfactant particle is a unilamellar or a multilamellar surfactant particle.

Item 39: The composition of any one of items 1 to 38, wherein the surfactant particle comprises a symmetry parameter (width to length ratio) of 1 or < 1, more preferably of 0.1 to 1, more preferably 0.5 to 1, most preferably 0.9 to 1.

Item 40: The composition of any one of items 1 to 39, wherein the surfactant particle comprises a zeta potential of at least 20 mV at pH 7, or of at least 30 mV at pH 7.

Item 41: The composition of any one of items 1 to 40, wherein the composition comprises particle sizes with a polydispersity index (PDI) of 0 to 1, more preferably 0.05 to 0.8, most preferably of 0.05 to 0.6.

Item 42: The composition of any one of items 1 to 41, wherein the composition comprises particle sizes with a polydispersity index (PDI) of <0.6.

Item 43: The composition of any one of items 1 to 42, wherein the molar amount of nitrogen atoms from Compound A in the composition to the molar amount of phosphorous atoms from the dsRNA (N/P) of N/P > 1; preferably of N/P = 1 to 5, most preferably of N/P = 1 to 3.5.

Item 44: The composition of any one of items 1 to 43, wherein the composition comprises a detection agent, preferably wherein the detection agent is a molecule or moiety that can be detected via a UV, fluorescence, luminescence and/or radioactive, mass signal.

Item 45: The composition of claim 44, wherein the detection agent is covalently bound to the dsRNA.

Item 46: The composition of any one of claims 2 to 45, wherein Compound A, Compound B and Compound C have a molar ratio of A:B:C of (35-65):(15-35):(15-35), preferably (40-60):(20-30):(20-30),

Item 47: The composition of any one of claims 2 to 45, wherein Compound A, Compound B and Compound C have a molar ratio of A:B:C of (40-60):(20-30):(20-30), preferably (45-55):(20-30):(20-30), preferably (48-52):(23-27):(23-27).

Item 48: The composition of any one of claims 1 to 47, wherein the dsRNA is a sequence that comprises 20 to 2000 base pairs, preferably wherein the dsRNA is a sequence that comprises 100 to 1000 base pairs, more preferably 400 to 600 base pairs.

Item 49: The composition of any one of claims 1 to 48, wherein the dsRNA is a siRNA, shRNA and/or a miRNA.

Item 50: The composition of any one of claims 1 to 49, wherein the gene silencing is RNAi.

Item 51: The composition of any one of claims 5 to 50, wherein the mineral agent is comprises a cation selected from group consisting of $Li^+$, $Na^+$, $K^+$, $Mg^{2+}$, $Ca^{2+}$, $Fe^{2+}$, $Fe^{3+}$, $Zn^{2+}$, $Ni^+$, $Ni^{2+}$, $Cu^+$, and $Cu^{2+}$ and/or comprises an anion selected from the list consisting of $F^-$, $Cl^-$, Br, $I^-$, $PO_4^{3-}$, $HPO_4^{2-}$,$H_2PO4^-$, $SO_3^{2-}$, $SO_4^{2-}$, formiate, acetate and propionate anions

Item 52: The composition of any one of claims 1 to 51, wherein the composition further includes one or more of

(i) a thickener;

(ii) a formulation adhesive; and/or

(iii) a freeze-drying stabilizer.

Item 53: The composition of any one of claims 1 to 52, wherein the composition is a specific pest control composition against a target arthropod.

Item 54: The composition of any one of claims 1 to 53, wherein the composition is formulated for spray application, foliar application, granular application, pouring application, drip application, broadcast application, aerial application, electrostatic application or injection application.

Item 55: The composition of any one of claims 1 to 54, wherein the arthropod is an organism of the order *Diptera, Hemiptera, Blattodea, Orthoptera, Coleoptera, Thysanoptera, Hymenoptera,* and/or *Lepidoptera, Phthiraptera.*

Item 56: The LNP composition of any one of claims 1 to 55, wherein the arthropod belongs to the family of *Culicidae,* preferably wherein the arthropod belongs to the genus of *Aedes,* most preferably wherein the arthropod belongs to species of *Aedes albopictus.*

**Item 57: A use of a composition** for the delivery of a dsRNA to an arthropod, wherein the composition comprises a surfactant particle with an average hydrodynamic diameter of 20 to 200 nm as determined by dynamic light scattering, wherein the surfactant particle encompasses a double-stranded RNA (dsRNA), wherein the surfactant particle comprises
Compound A with the structure (A-I) or (A-II);

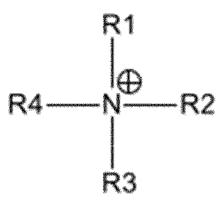

(A-I),

wherein

$R^1$ is selected from the group consisting of -H, or a linear or branched $C_{1-24}$ alkyl, $C_{2-24}$ alkenyl and $C_{2-24}$ alkynyl group, and

$R^2$, $R^3$ and $R^4$ are each independently selected from the group consisting of a linear or branched $C_{1-24}$ alkyl, $C_{2-24}$ alkenyl and $C_{2-24}$alkynyl group; or

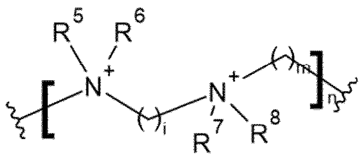

(A-II),

wherein

$R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;

i and m are each independently selected from 1 to 24; and

n is 1 to 1000; wherein

the use comprises a step of oral ingestion of the composition by the arthropod;

preferably wherein the composition is a composition according to any one of items 1 to 57.

Item 58: The use of item 57, wherein the dsRNA is a dsRNA for gene silencing, preferably wherein the gene silencing is RNAi.

Item 59: The use of item 57 or 58 wherein the composition of the invention is applied to a plant tissue, plant organ, plant epidermis, or any other part of a plant, preferably wherein the composition is applied to the leaf of a plant.

Item 60: The use of any one of claims 59, wherein the step of oral ingestion by the arthropod is subsequent to a step of applying the composition to the plant, preferably wherein the composition is applied to the plant by spray application.

Item 61: The use of claim 57 to 60, wherein the use comprises a step of transport of the surfactant particle through the arthropod digestive system.

Item 62: The use of any one of items 57 to 62, wherein the arthropod is an organism of the order *Diptera, Hemiptera, Blattodea, Orthoptera, Coleoptera, Thysanoptera, Hymenoptera,* and/or *Lepidoptera, Phthiraptera,* preferably wherein the arthropod is a pest insect and/or a vector insect.

Item 63: A method of manufacturing the composition according to any one of claims 2 to 56, wherein the method comprises mixing a solution 1 comprising Compound A according to any one of items 1 to 56, Compound B according to any one of items 2 to 56 and Compound C according to any one of items 2 to 56 with a solution 2 comprising the dsRNA according to any one of items 1 to 56, preferably wherein the solution 1 comprises an organic solvent such as ethanol and/or wherein the solution 2 is an aqueous solution.

Item 64: The method of item 64, wherein the mixing is conducted in a microfluidic reactor.

BRIEF DESCRIPTION OF THE FIGURES AND SEQUENCES

The figures show:

**Figure 1:** schematic diagram showing a ternary phase diagram for producing various LNP arthropod control compositions with varying components A, B, and C ratios.

**Figure 2:** cryogenic transmission electron microscopic (cryoTEM) image of a representative LNP arthropod control

composition (18.5-2, containing sequence 2) showing spherical and non-spherical particle morphology with particle sizes ranging from approximately 20 to 200 nm. Loaded dsRNA LNP particles appear lamellar structured and with higher contrast, and empty liposomes can be identified by their lipid bilayer structures (arrow marked "1"). Spherical dsRNA LNPs show a symmetry parameter of approximately 1 (arrows marked "2") and non-spherical dsRNA LNPs show symmetry parameters < 1, ranging from approximately 0.5 to 0.75 indicate (arrows marked ("3"))

**Figure 3:** image of electrophoresis results showing the complexation (top) and decomplexation (bottom) via ionic surfactant (SDS) of dsRNA (~500 bp, sequence 1) using indicated compositions No. 13-1, 18-1, 18.5-1, 19-1 (left) and compositions No. 13'-1, 18'-1, 18.5'-1, 19'-1 (right).

**Figure 4:** image of electrophoresis results showing the stability via complexation (top) and decomplexation (bottom) of LNP arthropod control composition No. 13-1, 18-1, 18.5-1, 19-1 and No. 13'-1, 18'-1, 18.5'-1, 19'-1 against the non-ionic surfactant Silwet Gold at 1 vol. %. The experiment indicates that these LNP compositions are stable against said surfactant.

**Figure 5:** image of electrophoresis results showing the stability via complexation (top) and decomplexation (bottom) of LNP arthropod control composition No. 13-1, 18-1, 18.5-1, 19-1 (left) and No. 13'-1, 18'-1, 18.5'-1, 19'-1 (right) against the non-ionic surfactant Ecosurf EH-3 at 1 vol. %. The experiment indicates that these LNP compositions are stable against said surfactant.

**Figure 6:** stereo microscopic images of sugar beet leaf disks (diameters =16 mm) with 5 μl droplets of a) water, b) pest control composition No. 18.5-2, and various wetting agents including c) N,N'-Dimethyltetradecylamine (+ DMTDA) (0.1.vol.%), d) CTAB (0.1 wt.%), e) Ecosurf EH-3 (0.1 vol.%) and f) Silwet Gold (0.1 vol.%).

**Figure 7:** (A) image of electrophoresis results showing the stability of LNP arthropod control composition (No. 18.5-1 and 18.5'-1) for the protection of dsRNA against RNase III. The LNP arthropod control composition 18.5 and 18.5' and dsRNA are intact after 0.5h incubation time. (B) image of electrophoresis results showing the stability of LNP arthropod control composition (No. 18.5-2) against various pH values ranging from pH 2 to pH 12 after 24 h incubation time. (C) image of electrophoresis results showing the shelf-life stability of at least 2 months of LNP arthropod control composition (No. 18.5-2).

**Figure 8:** fluorescence micrographs of *Aedes albopictus* larvae fed with Cy3-covalently labelled dsRNA as not-formulated in water, not-formulated in 1 vol% ethanolic solution and formulated as Cy3-dsRNA LNP arthropod control composition (according to No. 18.5-2), respectively. Bright field and fluorescence images were taken separately, then merged. Weak fluorescence signals appear in the digestive systems in all samples. However, a distinctive fluorescence signal appears only using the pesticide composition 18.5-2 in the head region inside the larvae after 1 h of feeding and remains there for at least 2 h during the fresh water treatment (indicated by a black circle).

EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

**Example 1: Preparation and Characterization of LNP-compositions comprising dsRNA**

The inventors demonstrate the manufacturing of LNP compositions in the phase diagram of **Fig. 1** by a microfluidic mixing process, as described for the preparation of the_LNP arthropod control compositions 18.5 and 18.5' below. This mixing process can generally be conducted by simple organic injection techniques using a variety of mixing reactor designs, mixing pumps or continuous flow mixing processes. These techniques are well-known to a person skilled in the art. A person skilled in the art is able to control the particle size distribution, size, structure and morphology of the resulting LNP particles by controlling the mixing process, for example by adjusting the mixing parameters using a mixing chip. As representative nucleic acid, dsRNA was used as model in all experiments. The LNP arthropod control compositions is formed by a charge interaction of the negatively charged nucleic acid and positively charged lipids and additional uncharged lipid components.

Preparation of LNP arthropod control compositions 18.5 and 18.5': The inventors manufactured LNP-compositions containing dsRNA by mixing a lipid-containing organic phase and a nucleic acid-containing water phase. The organic phase contains 0.569 g/L didodecyldimethylammonium bromide (DDAB, lipid A) or 0.776 g/L dimethyldioctadecylammonium bromide (DODAB, lipid A') respectively, 0.591 g/L Kolliphor HS-15 (lipid B), and 0.458 g/L lecithin from egg yolk (lipid C) representing a molar ratio of 50:25:25 (Lipid A or A':lipid B:lipid C) in ethanol. The lipid solution was mixed with an aqueous phase containing dsRNA of either a non-active control dsRNA (samples labelled "-1", with a length of 500 base pairs) or a dsRNA designed with potential activity (samples labelled "-2", with a length of 500 base pairs) at a concentration of 0.222 g/l using in a microfluidic process. Continuous microfluidic mixing was achieved using a microfluidic chip (μ-slide III 3in1, Ibidi, Thermo Fisher: 17296163) that was connected to two solvent delivery systems (Prepstar SD-1, Varian) via tubing (OD 3 mm, ID 2 mm). The solvent delivery systems were connected to reservoirs of ethanolic and aqueous solutions via tubing (OD 3 mm, ID 2 mm). The solutions were kept constant at 65°C during the microfluidic mixing process. The total flow rate was set at 50 mL/min using a flow rate ratio of the aqueous to ethanolic solutions of 45 mL/min to 5 mL/min. By using this flow rate ratio, a final dsRNA concentration of 0.2 mg/mL in a 10 vol.% ethanolic solution was achieved. The LNP arthropod control composition was collected and stored in polypropylene tubes at 25 °C.

In these experiments, the lecithin from egg-yolk was composed as indicated in the table below:

| | |
|---|---|
| Phosphatidylcholine | 64.0 - 79.0 % |
| Phosphatidylethanolamine | 12.0 - 18.0 % |
| Lysophosphatidylcholine | ≤3 % |
| Sphingomyelin | ≤3 % |
| Water | ≤2 % |
| Cholesterol | ≤1 % |
| Peroxide value | ≤3 meq/kg |
| Acid value | ≤25 mg KOH/g |
| Iodine value | ≥64 |

Measurement of the particle size and zeta potential of the LNP arthropod control compositions: The particle size distribution and zeta potential of the LNP dsRNA was measured using a Zetasizer Nano ZS (Malvern Panalytical) with DTS1070 cells (Malvern Panalytical). For the particle size distribution, polydispersity index and zeta potential measurements machine setting were set at:

- reflective index (RI) = 1.330
- viscosity (cP) = 0.8872
- measurement position = 5.5 mm and attenuator to either 6 or 7 (particle size distribution, PDI).
- measurement position = 2.0 mm and attenuator to either 9 or 10 (zeta potential).

Each measurement was done at 25°C with an initial equilibration time of 0.5 min and 50 scans. Acquired data was processed using the software Zetasizer v7.10.

LNP compositions comprising the properties indicated in Table 1 were obtained, with Component A being DDAB (No. 13, 18, 18.5, 19, 25) or DODAB (No. 13', 18', 18.5', 19', 25'), using a non-active control dsRNA sequence

(500 base pairs length, samples labelled "-1") dsRNA sequence designed with potential activity (500 base pairs length, samples labelled "-2",); Component B being Kolliphor HS 15 and Component C being lecithin from egg yolk.

Table 1: Composition and physicochemical properties of various LNP-compositions

| **Composition No.** | **dsRNA Sequence** | **N/P ratio (-)** | **A (mol. %)** | **B (mol. %)** | **C (mol. %)** | **d (nm)** | **PDI (-)** | **Zeta-potential (mV)** |
|---|---|---|---|---|---|---|---|---|
| 13-1 | non-active control dsRNA | 2.4 | 60 | 20 | 20 | 90 | 0.22 | 42.8 |

(continued)

| Composition No. | dsRNA Sequence | N/P ratio (-) | A (mol. %) | B (mol. %) | C (mol. %) | d (nm) | PDI (-) | Zeta-potential (mV) |
|---|---|---|---|---|---|---|---|---|
| 13-2 | dsRNA designed with potential activity | 2.4 | 60 | 20 | 20 | 88 | 0.29 | 39.3 |
| 13'-1 | non-active control dsRNA | 2.4 | 60 | 20 | 20 | 104 | 0.24 | 37.9 |
| 13'-2 | dsRNA designed with potential activity | 2.4 | 60 | 20 | 20 | 92 | 0.33 | 41.7 |
| 18-1 | non-active control dsRNA | 2.0 | 50 | 20 | 30 | 103 | 0.23 | 33.6 |
| 18-2 | dsRNA designed with potential activity | 2.0 | 50 | 20 | 30 | 88 | 0.27 | 38.0 |
| 18'-1 | non-active control dsRNA | 2.0 | 50 | 20 | 30 | 133 | 0.22 | 39.5 |
| 18'-2 | dsRNA designed with potential activity | 2.0 | 50 | 20 | 30 | 87 | 0.32 | 46.3 |
| 18.5-1 | non-active control dsRNA | 2.0 | 50 | 25 | 25 | 114 | 0.25 | 35.5 |
| 18.5-2 | dsRNA designed with potential activity | 2.0 | 50 | 25 | 25 | 83 | 0.31 | 44.6 |
| 18.5'-1 | non-active control dsRNA | 2.0 | 50 | 25 | 25 | 148 | 0.19 | 32.7 |
| 18.5'-2 | dsRNA designed with potential activity | 2.0 | 50 | 25 | 25 | 112 | 0.36 | 29.7 |
| 19-1 | non-active control dsRNA | 2.0 | 50 | 30 | 20 | 106 | 0.21 | 37.6 |
| 19-2 | dsRNA designed with potential activity | 2.0 | 50 | 30 | 20 | 82 | 0.26 | 49.5 |
| 19'-1 | non-active control dsRNA | 2.0 | 50 | 30 | 20 | 140 | 0.20 | 46.9 |
| 19'-2 | dsRNA designed with potential activity | 2.0 | 50 | 30 | 20 | 86 | 0.32 | 47.1 |
| 25-2 | dsRNA designed with potential activity | 1.6 | 40 | 30 | 30 | 93 | 0.22 | 39.7 |
| 25'-2 | dsRNA designed with potential activity | 1.6 | 40 | 30 | 30 | 127 | 0.26 | 32.6 |

Examination of the Shape of the LNPs containing dsRNA via cryoTEM: The inventors further investigated the shape of the LNPs using cryoTEM. Exemplary results are shown for a representative LNP arthropod control composition (18.5-2) in **Fig. 2.**

**Example 2 Stability of LNP compositions comprising dsRNA.**

Agarose gel electrophoresis and SDS decomplexation: Agarose gel electrophoresis was used to investigate the complexation and decomplexation ability and the stability of LNP arthropod control compositions. In these experiments, the inventors examined various LNP compositions of Table 1.

A 2% agarose gel was used for gel electrophoresis. For this purpose, 0.9 g agarose (Agarose NEEO Ultra-Quality - Carl Roth - Order No. 2267.4) was mixed with 45 ml buffer (1x TAE buffer Tris-acetate-EDTA, Thermo Fisher, Catalog

number: B49) and heated in a microwave for approximately 90 s at 700 W until a clear solution was obtained. The hot solution was poured into a casting frame to cool down and solidify. The gel was then placed in a gel chamber, which was filled with 500 mL of running buffer (1x TAE buffer Tris-acetate-EDTA, Thermo Fisher, Catalog number: B49). The comb was then removed, creating wells with a volume of approximately 10-15 μL. The samples for agarose gel electrophoresis were prepared by mixing dsRNA or LNP dsRNA (8 μL, 0.2 μg/μL dsRNA) with 1 μl loading dye (10x BlueJuice ThermoFisher, 10816015) and 1 μl SYBR Gold (3x in DMSO, ThermoFisher, S11494).

The decomplexation of the LNP arthropod control compositions was achieved by adding 1 μL of 0.2 wt.% SDS (ThermoFisher, 28312). The gel was loaded with 8 μL of the LNP dsRNA formulations or 9 μL of the decomplexed samples respectively and then run for 45 min. at a voltage of 80mV (Biorad - PowerPac™ Basic Power Supply). The gel was then investigated using a UV chamber at appropriate excitation wavelength of the used dye (Biorad - Gel Doc XR+ Gel Documentation System). 3 μl of a 100 bp DNA marker (100 bp DNA-Ladder, ThermoFisher, Cat. No.15628050) was mixed with 1 μl 3x Sybr Gold and also loaded to the gel in order to check for correct size.

Addition of the negatively charged surfactant SDS resulted in the decomplexation of the previously stable LNPs of the invention (compare **Fig. 3**). A band corresponding to the decomplexed samples was detected in all samples after decomplexing with SDS indicated a successful loading of all LNPs.

Additive stability assay: The stability of various of the above LNP arthropod control compositions against wetting agents were investigated using agarose gel electrophoresis. 5 mL of LNP arthropod control compositions were mixed with either 0.05 mL (1.0 vol.%) or 0.005 mL (0.1 vol.%) of wetting agents using Silwet Gold (Spiess-Urania Chemicals GmbH, 5310-00) (results shown in **Fig. 4**) or Ecosurf EH-3 (Sigma Aldrich, STS0015) (results shown in **Fig. 5**) respectively. Samples were incubated at room temperature and analyzed after 24h by agarose gel electrophoresis according to the same procedure described above. Addition of negatively charged SDS resulted in the decomplexation of the LNPs of the invention. The experiments demonstrate a high compatibility of the LNP-compositions with these additives.

Wetting Experiments: The inventors investigated the influence of various wetting agents on the wetting behavior of the LNP compositions on sugar beet leaf disks. The tested wetting agents included ionizable wetting agents (such as the tertiary amine N,N'-Dimethyltetradecylamine (DMTDA)) and cationic wetting agents (such as the quaternary amine Cetyltrimethylammonium bromide (CTAB)), as well as several commercially available wetting agents (Ecosurf EH 3 and Silwet Gold). Addition of all tested wetting agents led to a decrease in the surface angle of the LNP-compositions on the sugar beet leaf disks (see **Fig. 6**). In the following, the inventors focused on the wetting agents with the strongest wetting effect, namely Ecosurf EH 3 and Silwet Gold.

RNase III stability assay: The ability of the LNP arthropod control compositions to provide protection of the dsRNA molecules against degradation was tested in an *in vitro* RNase assay. Therefore, formulated (LNP arthropod control composition No. 18.5-1) or non-formulated dsRNA (20 μL, 0.2 μg/μL) was mixed with ultra-pure water (29 μL) and ShortCut RNase III (1 μL, 2 U/μL, New England Biolabs, M0245S). Reactions were incubated at 37°C for 30 min and quenched by adding proteinase K (5 μL, 0.8 U/μL, New England Biolabs, P8107S) and incubated at 37°C for another 30 min. Results were investigated using agarose gel electrophoresis according to the procedure described above. The results are shown in **Fig. 7A.** The samples, in which the dsRNA was formulated in the LNP compositions, did not demonstrate RNA degradation, whereas the dsRNA control sample without LNPs was degraded by the RNase. Addition of negatively charged SDS resulted in the decomplexation of the LNPs of the invention. After SDS decomplexation, the samples with the loaded-LNP nanoparticles and the samples with the loaded-LNP nanoparticles and addition of RNaseIII show a high conformity indicating that the LNP composition protected the dsRNA against degradation. This demonstrates the protective properties of the LNPs on the dsRNA.

pH stability assay: The ability of the LNP arthropod control compositions to provide protection of the dsRNA molecules against high and low pH values was tested in an *in vitro* pH value assay. Therefore, formulated (LNP arthropod control composition No. 18.5-2) or non-formulated dsRNA (50 μL, 0.2 μg/μL) was mixed with an aqueous solution (25 μL) adjusted to pH 2, 3, 4 using 1M HCl or pH 10, 11, 12 using 1M NaOH. Reactions were incubated at room temperature for 24h and were subsequently buffered by addition of 0.1M ammonium acetate (25 μL). The results were investigated using agarose gel electrophoresis according to the same procedure described above. The results are shown in **Fig. 7B.**

Shelf-life stability: The inventors tested the shelf-life ot the LNP arthropod control composition 18-2. In these experiments, they stored said composition for 2 months at room temperature under sunlight. Over this time, the composition did not show any decomplexation. Furthermore, the inventors were able to decomplexate the dsRNA by subsequent addition of SDS (compare **Fig. 7C**)

**Example 3: Uptake and stability of LNP-compositions comprising dsRNA in mosquito larvae**

Fluorescence microscopy was used to investigate the uptake and stability of formulated and non-formulated fluorescently labeled dsRNA in mosquito larvae (Aedes albopictus). The dsRNA (active dsRNA) was covalently labeled using a Cy3 labeling kit according to the manufacturer's protocol (Mirus Bio, Label IT Nucleic Acid Labeling Kit, Cy3). The labeled dsRNA was formulated according to the above-described procedure to obtain an LNP arthropod control

composition according to the compositions 18.5-2 in Table 1. Then 2.5 µl of said Cy3-dsRNA LNP with a dsRNA concentration of 0.1 mg/ml was added to 22.5 µl of tap water.

The non-formulated, labeled Cy3-dsRNA reference samples were prepared accordingly by adding 2.1 µl (0.12 mg/ml) to 22.9 µl of water and 2.1 µl to 22.9 µl of an ethanolic solution (1 vol%). The larvae (stage 1, hatched within 24h) were fed in these feeding solutions for 1 h. The feeding solutions were then removed and the larvae were transferred to fresh 25 µl water for up to 2 h. Both microscopic brightfield images (Leica DM5000B, 4x objective) and fluorescence images (Leica EL6000 light source, Leica fluorescence filter system N3 ET) were taken after 1 h incubation in feeding solution and subsequently after 1 and 2 h post fresh water treatment. Brightfield and fluorescence images were merged using the Leica software LAS v4.13.

The results are shown in **Fig. 8.**

## Claims

1. A composition, comprising a surfactant particle with an average hydrodynamic diameter of 20 to 200 nm as determined by dynamic light scattering, wherein the surfactant particle encompasses a double-stranded RNA (dsRNA) for gene silencing against an arthropod target gene, wherein the surfactant particle comprises

   Compound A with the structure (A-I) or (A-II);

   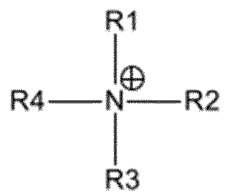

   (A-I),

   wherein
   $R^1$ is selected from the group consisting of -H, or a linear or branched $C_{1-24}$ alkyl, $C_{2-24}$ alkenyl and $C_{2-24}$ alkynyl group, and
   $R^2$, $R^3$ and $R^4$ are each independently selected from the group consisting of a linear or branched $C_{1-24}$ alkyl, $C_{2-24}$ alkenyl and $C_{2-24}$alkynyl group;
   or

   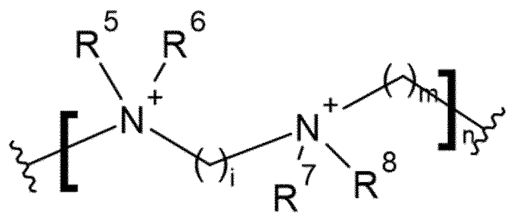

   (A-II),

   wherein
   $R^5$, $R^6$, $R^7$ and $R^8$ are each independently selected from the group consisting of a linear or branched $C_{1-24}$ alkyl, $C_{2-24}$ alkenyl and $C_{2-24}$ alkynyl group;
   i and m are each independently selected from 1 to 24; and
   n is 1 to 1000;
   preferably, wherein Compound A comprises the structure (A-I),

   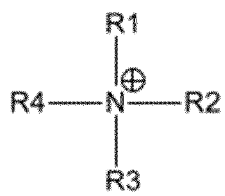

   (A-I),

wherein $R^1$ is selected from the group consisting of -H, or a linear or branched $C_{1-24}$ alkyl, $C_{2-24}$ alkenyl and $C_{2-24}$ alkynyl group, and
$R^2$, $R^3$ and $R^4$ are each independently selected from the group consisting of a linear or branched $C_{1-24}$ alkyl, $C_{2-24}$ alkenyl and $C_{2-24}$alkynyl group.

**2.** The composition of claim 1, wherein the surfactant particle is a lipid nanoparticle, preferably a liposome;

and/or wherein the surfactant particle additionally comprises

(i) Compound B, wherein Compound B is a non-ionic lipid; and/or
(ii) Compound C, wherein Compound C comprises an amphoteric phospholipid;

preferably wherein the surfactant particle comprises Compound B and Compound C.

**3.** The composition of claim 1 or 2, wherein the composition, preferably the surfactant particle, additionally comprises a wetting agent D, wherein the wetting agent D is selected from the group consisting of a non-ionic surfactant, a cationizable surfactant and a cationic surfactant;
preferably wherein the non-ionic surfactant is selected from the group consisting of an ethoxylated and/or propoxylated alcohol; and a polyether modified trisiloxane.

**4.** The composition of any one of claims 1 to 3, wherein

$R^1$ and $R^2$ are each independently selected from the group consisting of a $C_{1-6}$ alkyl, $C_{2-6}$ alkenyl and $C_{2-6}$ alkynyl group; and/or
$R^3$ and $R^4$ are each independently selected from the group consisting of a $C_{12-24}$ alkyl, a $C_{12-24}$ alkenyl and a $C_{12-24}$ alkynyl group;
preferably wherein Compound A is a didodecyldimethylammonium or dimethyldioctadecylammonium cation.

**5.** The composition of any one of claims 2 to 4, wherein Compound B comprises the structure (B-I)

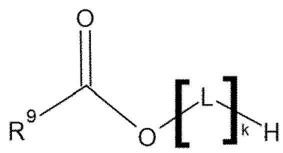
(B-I),

wherein

$R^9$ is selected from the group consisting of a $C_{1-24}$ alkyl, $C_{2-24}$ alkenyl and $C_{2-24}$ alkynyl group, wherein the $C_{1-24}$ alkyl, $C_{2-24}$ alkenyl, and $C_{2-24}$ alkynyl group is optionally substituted with one or more -OH;
$[L]_k$ is a polyoxyalkylene group comprising k repeating units, such as a polyoxymethylene group (referring to $[-(CH)_2-O-]_k$), a polyoxyethylene group (referring to $[-(C_2H_4)-O-]_k$), or a polyoxypropylene group (referring to $[-C_3H_6)-O-]_k$), or a copolymer of [L] comprising k oxyalkylene repeating units, preferably wherein the oxyalkylene repeating units are each independently selected from the group consisting of $[-(CH)_2-O-])$, $[-(C_2H_4)-O-]$ and $[-(C_3H_6)-O-]$;
wherein k is 1 to 10000.

**6.** The composition of any one of claims 2 to 5, wherein Compound C is egg-lecithin and/or wherein the amphoteric phospholipid comprises a compound selected from of the group consisting of a phosphatidylcholine, a phosphatidylethanolamine, lysophosphatidylcholine, and a sphingomyelin, preferably wherein the phosphatidylcholine comprises the formula (C-I),

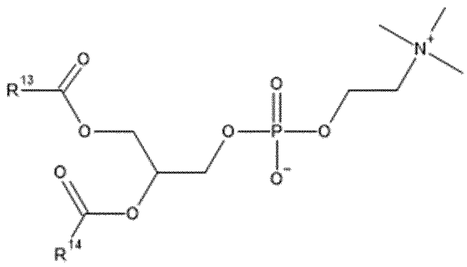

(C-I),

wherein $R^{13}$ and $R^{14}$ are each independently selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group; and/or
wherein the phosphatidylethanolamine comprises the formula (C-II),

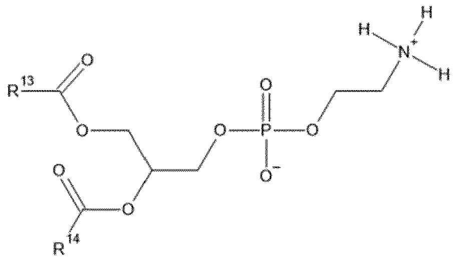

(C-II),

wherein $R^{13}$ and $R^{14}$ are each independently selected from the group consisting of a linear or branched $C_{10-24}$ alkyl, $C_{10-24}$ alkenyl and $C_{10-24}$ alkynyl group, preferably wherein $R^{13}$ and $R^{14}$ are each independently selected from the group consisting of a linear or branched $C_{12-20}$ alkyl and a $C_{12-20}$ alkenyl group.

**7.** The composition of any one of claims 3 to 6, wherein the wetting agent D is the non-ionic surfactant, preferably, wherein the ethoxylated and/or propoxylated alcohol comprises the structure (D-I)

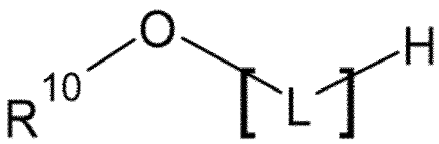

(D-I),

wherein $R^{10}$ is a linear or branched $C_{8-24}$ alkyl, $C_{8-24}$ alkenyl and $C_{8-24}$ alkynyl group,
[L] is a polyoxyalkylene group comprising k repeating units, such as a polyoxymethylene group (referring to $[-(CH)_2-O-]_b$), a polyoxyethylene group (referring to $[-(C_2H_4)-O-]_b$), or a polyoxypropylene group (referring to $[-C_3H_6)-O-]_b$), or a copolymer comprising k oxyalkylene repeating units, preferably wherein the oxyalkylene repeating units are independently selected from the group consisting of $[-(CH)_2-O-]$), $[-(C_2H_4)-O-]$ and $[-(C_3H_6)-O-]$;
and b is 1 to 10000; and/or
wherein the polyether-modified trisiloxane comprises the structure (D-II):

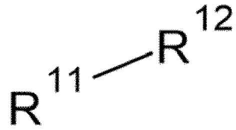

(D-II),

wherein
$R^{11}$ is a linear trisiloxane with the structure $R^{11'}{}_3Si-O-SiR^{11'}{}_2-O-SiR^{11'}{}_3$, or a cyclic trisiloxane with the structure $[O-SiR^{11'}{}_2]_3$, wherein each of the $R^{11'}$ is independently selected form the group consisting of $C_{1-24}$ alkyl, $C_{2-24}$ alkenyl

and $C_{2-24}$ alkynyl group,
$R^{12}$ is a substituent bound to $R^{11'}$ and is a polyether, such as a polyethylene glycol or a polypropylene glycol or a copolymer thereof, with 1 to 10.000 repeating units, optionally wherein the polyether is terminated with a methyl group.

**8.** The composition of any one of claims 3 to 6, wherein the wetting agent D is the cationizable surfactant and/or the cationic surfactant, preferably, wherein the cationizable surfactant is a tertiary amine-derivative and/or, wherein the cationic surfactant is a quaternary ammonium-derivative, more preferably wherein the tertiary amine-derivative comprises the structure(D-IIIb) and/or the quaternary ammonium-derivative comprises the structure (D-IIIa)

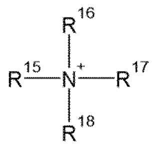 (D-IIIa), 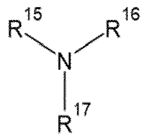 (D-IIIb)

wherein

$R^{18}$ is selected from the group consisting of a linear or branched $C_{1-24}$ alkyl, $C_{2-24}$ alkenyl and $C_{2-24}$ alkynyl group, and
$R^{15}$, $R^{16}$ and $R^{17}$ are each independently selected from the group consisting of a linear or branched $C_{1-24}$ alkyl, $C_{2-24}$ alkenyl and $C_{2-24}$ alkynyl group.

**9.** The composition of any one of claims 1 to 8, wherein the molar amount of nitrogen atoms from Compound A in the composition to the molar amount of phosphorous atoms from the dsRNA (N/P) of N/P > 1; preferably of N/P = 1 to 5, most preferably of N/P = 1 to 3.5.

**10.** The composition of any one of claims 2 to 9, wherein Compound A, Compound B and Compound C have a molar ratio of A:B:C of (35-65):(15-35):(15-35), preferably (40-60):(20-30):(20-30), preferably (45-55):(20-30):(20-30), preferably (48-52):(23-27):(23-27)

**11.** The composition of any one of claims 1 to 10, wherein the composition is a specific pest control composition against a target arthropod; and/or wherein the arthropod is an organism of the order *Diptera, Hemiptera, Blattodea, Orthoptera, Coleoptera, Thysanoptera, Hymenoptera,* and/or *Lepidoptera, Phthiraptera,* preferably the arthropod belongs to the family of *Culicidae,* preferably wherein the arthropod belongs to the genus of *Aedes,* most preferably wherein the arthropod belongs to species of *Aedes albopictus.*

**12. A use of a composition according to claim 1 to 11** for the delivery of a dsRNA to an arthropod, wherein the use comprises a step of oral ingestion of the composition by the arthropod, preferably wherein the dsRNA is a dsRNA for gene silencing, more preferably wherein the gene silencing is RNAi.

**13.** The use of claim 12, wherein the composition of the invention is applied to a plant tissue, plant organ, plant epidermis, or any other part of a plant, preferably wherein the composition is applied to the leaf of a plant, preferably wherein the step of oral ingestion by the arthropod is subsequent to the step of applying of the composition to the plant.

**14.** The use of claim 12 or 13, wherein the use comprises a step of transport of the surfactant particle through the arthropod digestive system.

**15. A method of manufacturing** the composition according to any one of claims 2 to 11, wherein the method comprises mixing a solution 1 comprising Compound A according to any one of claims 1 to 11, Compound B according to any one of claims 2 to 11 and Compound C according to any one of claims 2 to 11 with a solution 2 comprising the dsRNA according to any one of claims 1 to 11, preferably wherein the solution 1 comprises an organic solvent such as ethanol and/or wherein the solution 2 is an aqueous solution.

FIGURE 1

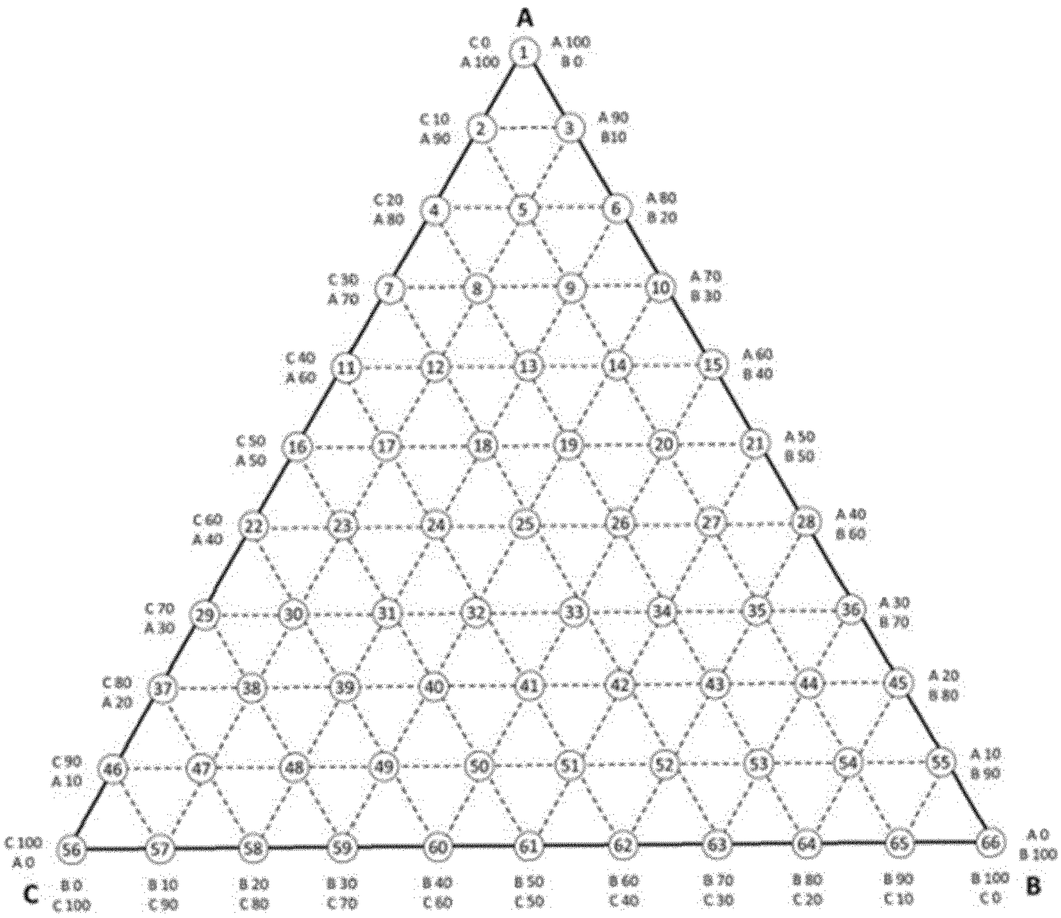
A
B
C
C 0 A 100
A 100 B 0
C 10 A 90
A 90 B10
C 20 A 80
A 80 B 20
C 30 A 70
A 70 B 30
C 40 A 60
A 60 B 40
C 50 A 50
A 50 B 50
C 60 A 40
A 40 B 60
C 70 A 30
A 30 B 70
C 80 A 20
A 20 B 80
C 90 A 10
A 10 B 90
C 100 A 0
A 0 B 100
B 0 C 100
B 10 C 90
B 20 C 80
B 30 C 70
B 40 C 60
B 50 C 50
B 60 C 40
B 70 C 30
B 80 C 20
B 90 C 10
B 100 C 0
1
2
3
4
5
6
7
8
9
10
11
12
13
14
15
16
17
18
19
20
21
22
23
24
25
26
27
28
29
30
31
32
33
34
35
36
37
38
39
40
41
42
43
44
45
46
47
48
49
50
51
52
53
54
55
56
57
58
59
60
61
62
63
64
65
66

FIGURE 2

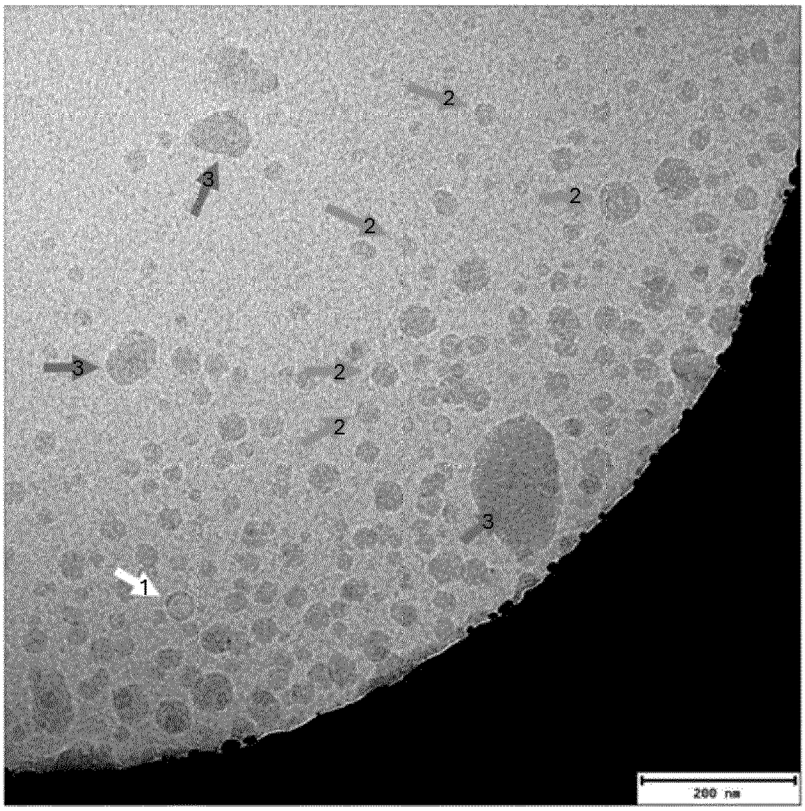

FIGURE 3

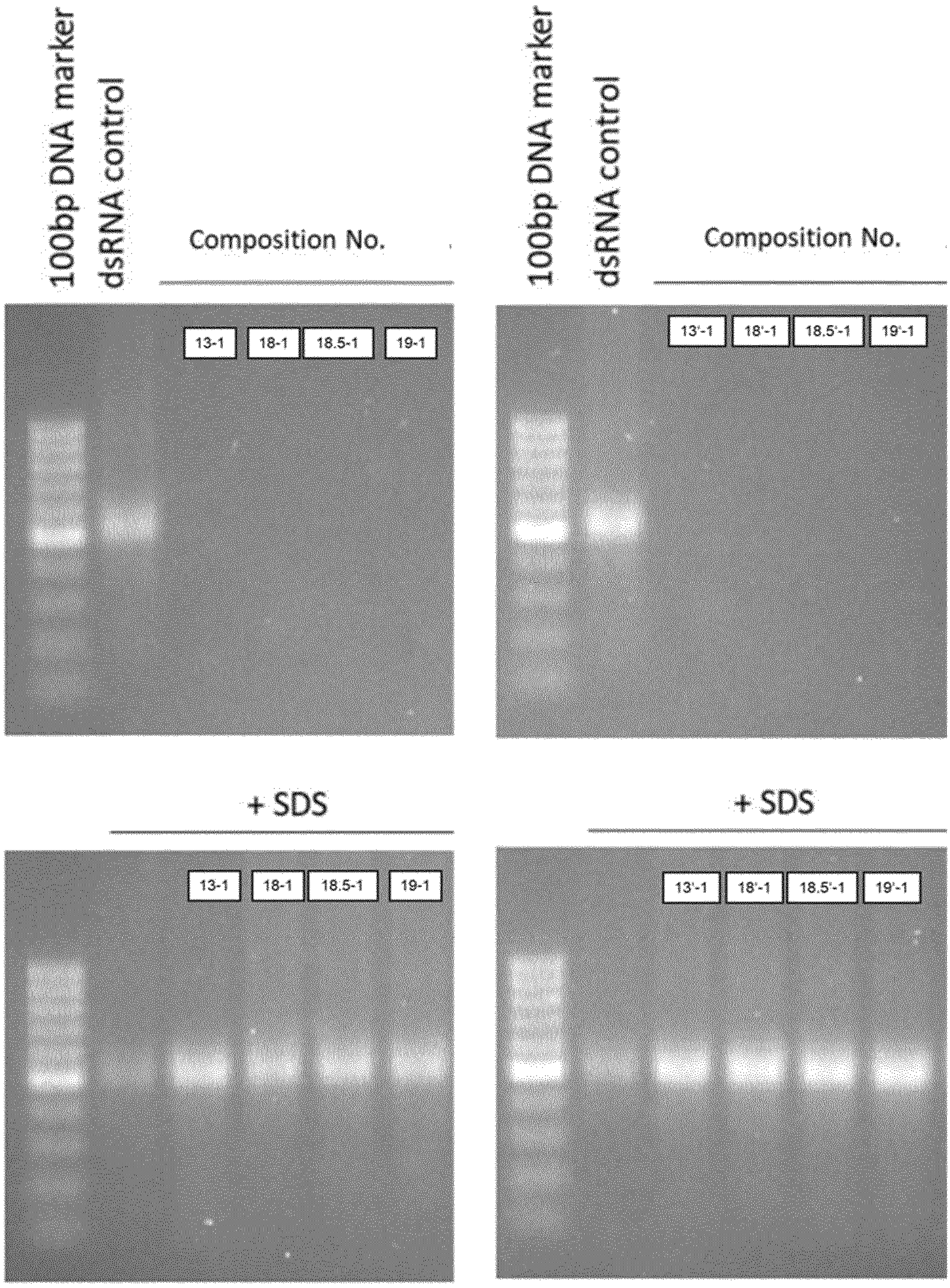
100bp DNA marker
dsRNA control
Composition No.
13-1
18-1
18.5-1
19-1
100bp DNA marker
dsRNA control
Composition No.
13'-1
18'-1
18.5'-1
19'-1
+ SDS
13-1
18-1
18.5-1
19-1
+ SDS
13'-1
18'-1
18.5'-1
19'-1

FIGURE 4

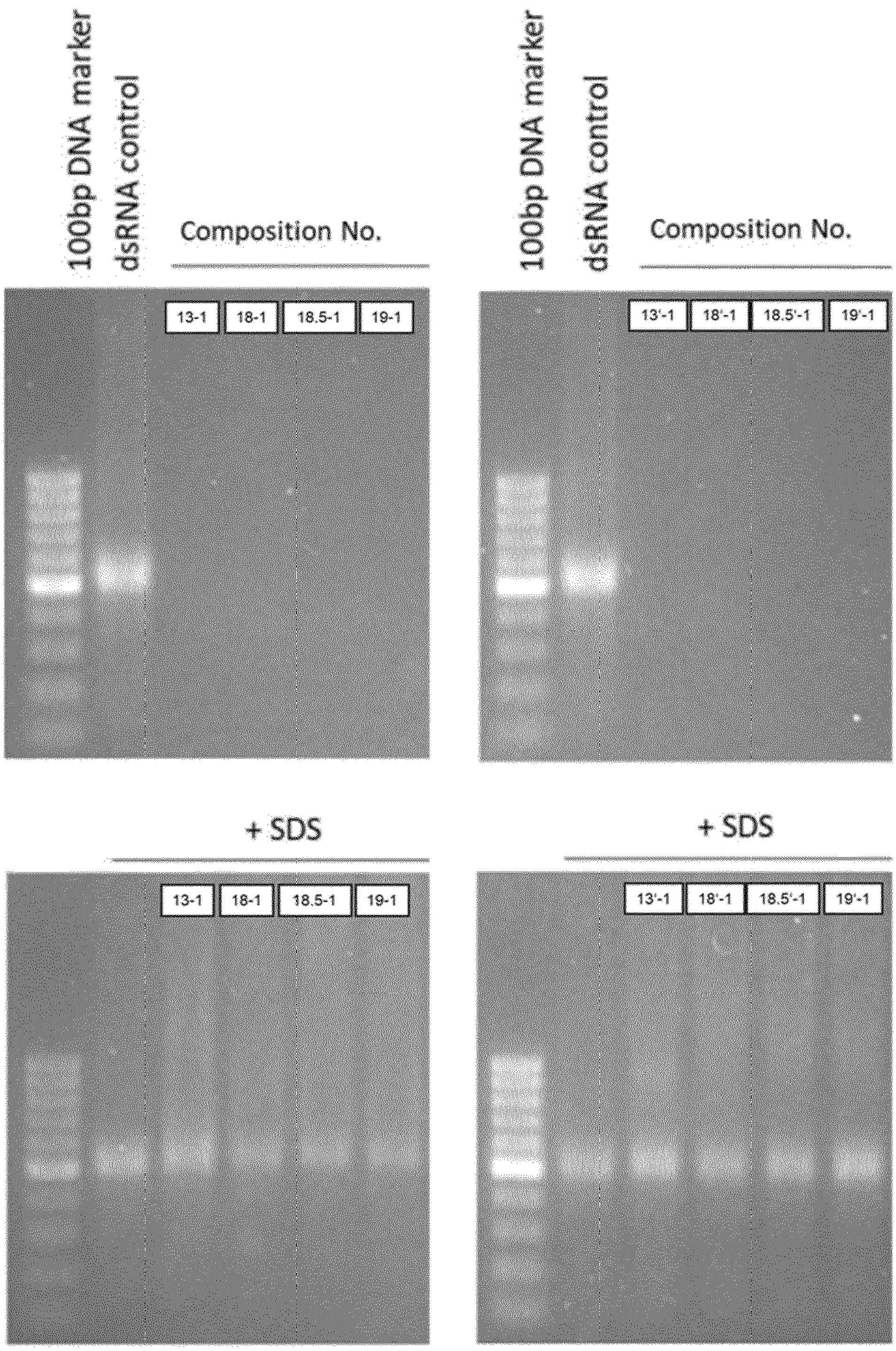
100bp DNA marker
dsRNA control
Composition No.
13-1
18-1
18.5-1
19-1
100bp DNA marker
dsRNA control
Composition No.
13'-1
18'-1
18.5'-1
19'-1
+ SDS
13-1
18-1
18.5-1
19-1
+ SDS
13'-1
18'-1
18.5'-1
19'-1

FIGURE 5

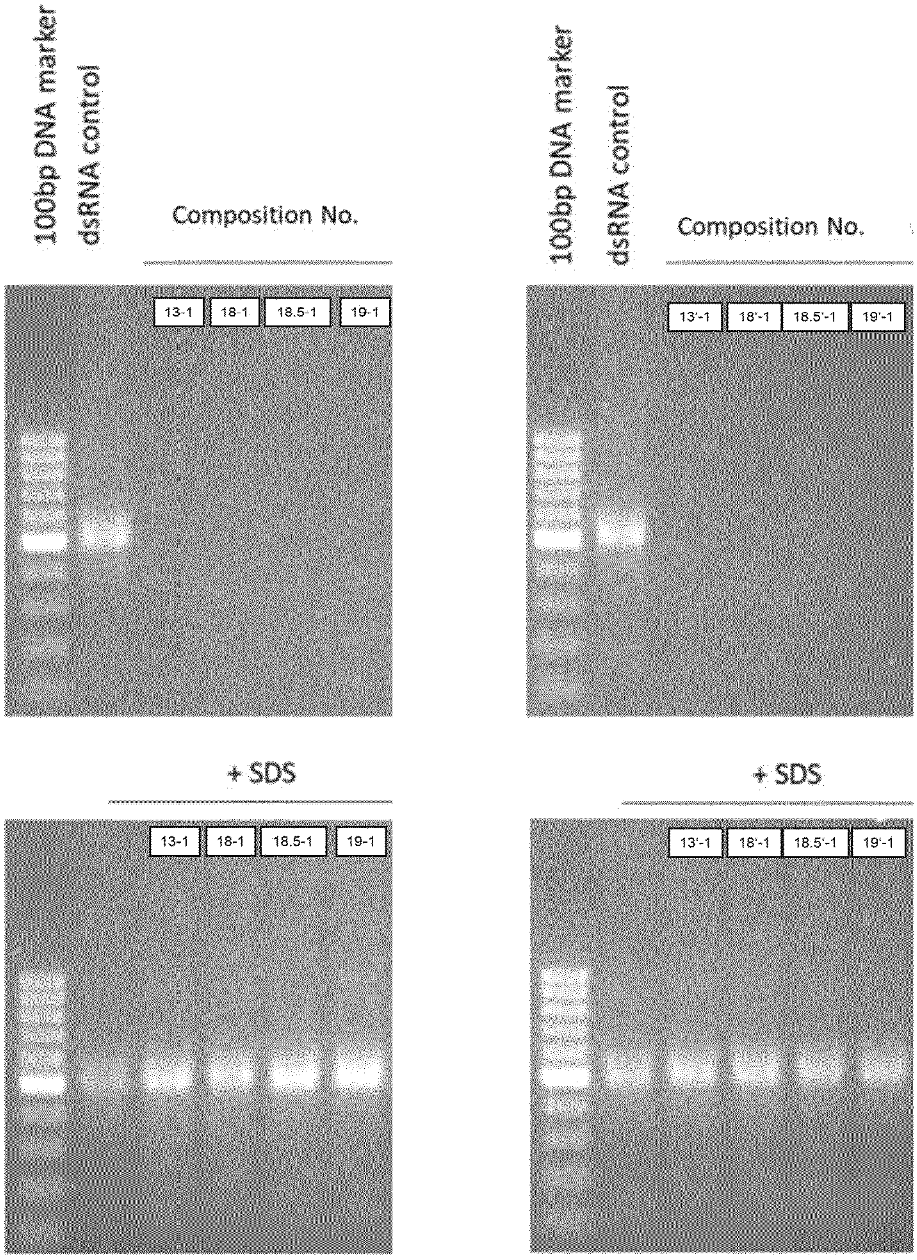
100bp DNA marker
dsRNA control
Composition No.
13-1
18-1
18.5-1
19-1
100bp DNA marker
dsRNA control
Composition No.
13'-1
18'-1
18.5'-1
19'-1
+ SDS
13-1
18-1
18.5-1
19-1
+ SDS
13'-1
18'-1
18.5'-1
19'-1

FIGURE 6

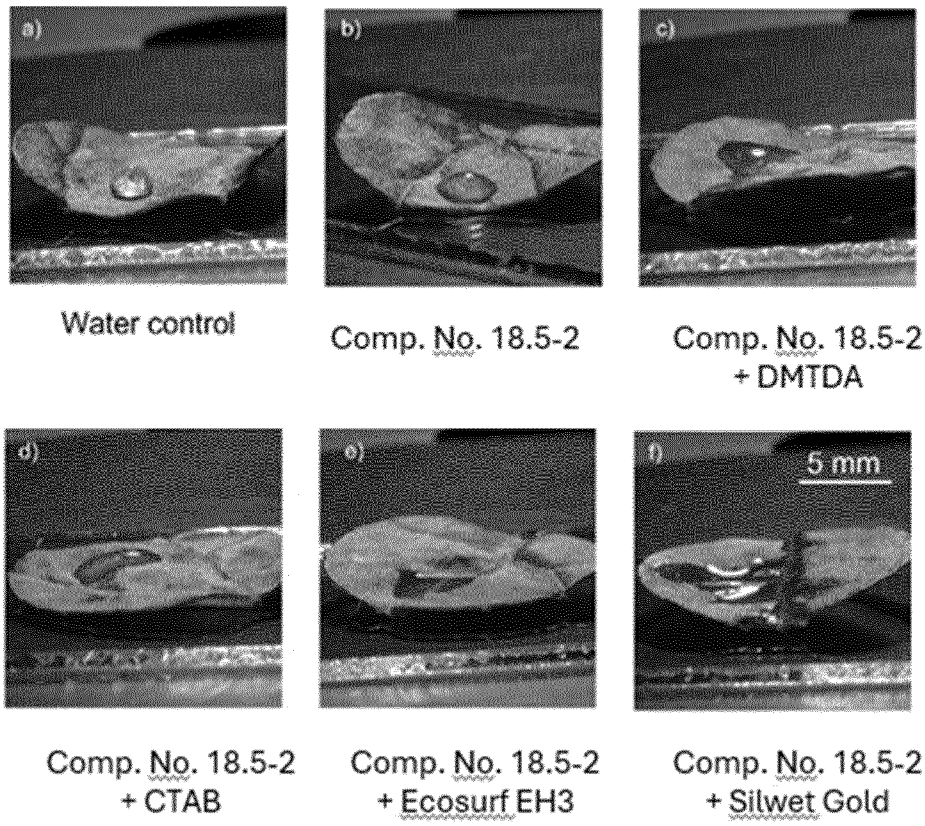
a)
b)
c)
Water control
Comp. No. 18.5-2
Comp. No. 18.5-2
+ DMTDA
d)
e)
f)
5 mm
Comp. No. 18.5-2
+ CTAB
Comp. No. 18.5-2
+ Ecosurf EH3
Comp. No. 18.5-2
+ Silwet Gold

FIGURE 7

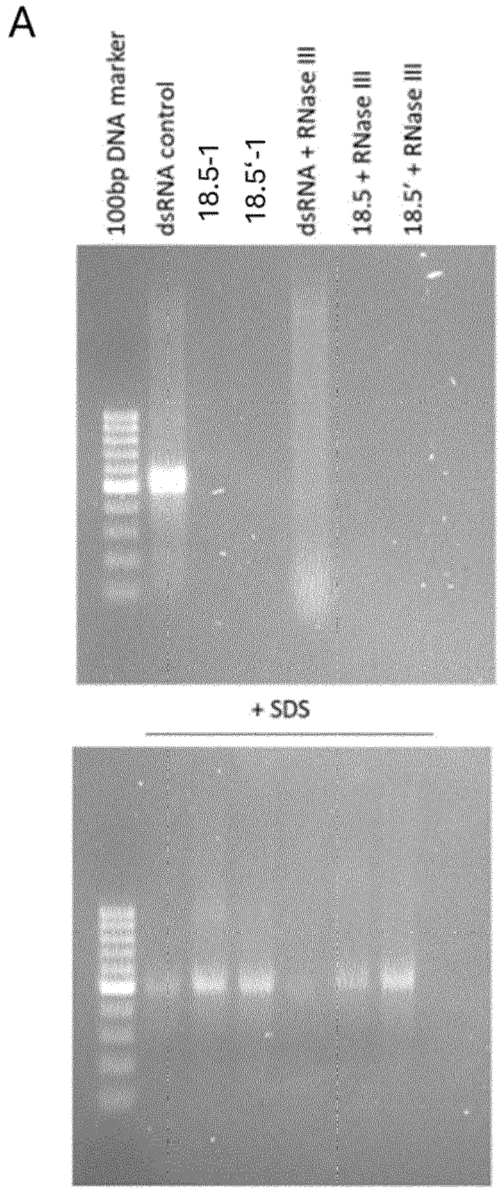
A
100bp DNA marker
dsRNA control
18.5-1
18.5'-1
dsRNA + RNase III
18.5 + RNase III
18.5' + RNase III
+ SDS

FIGURE 7 (cont.)

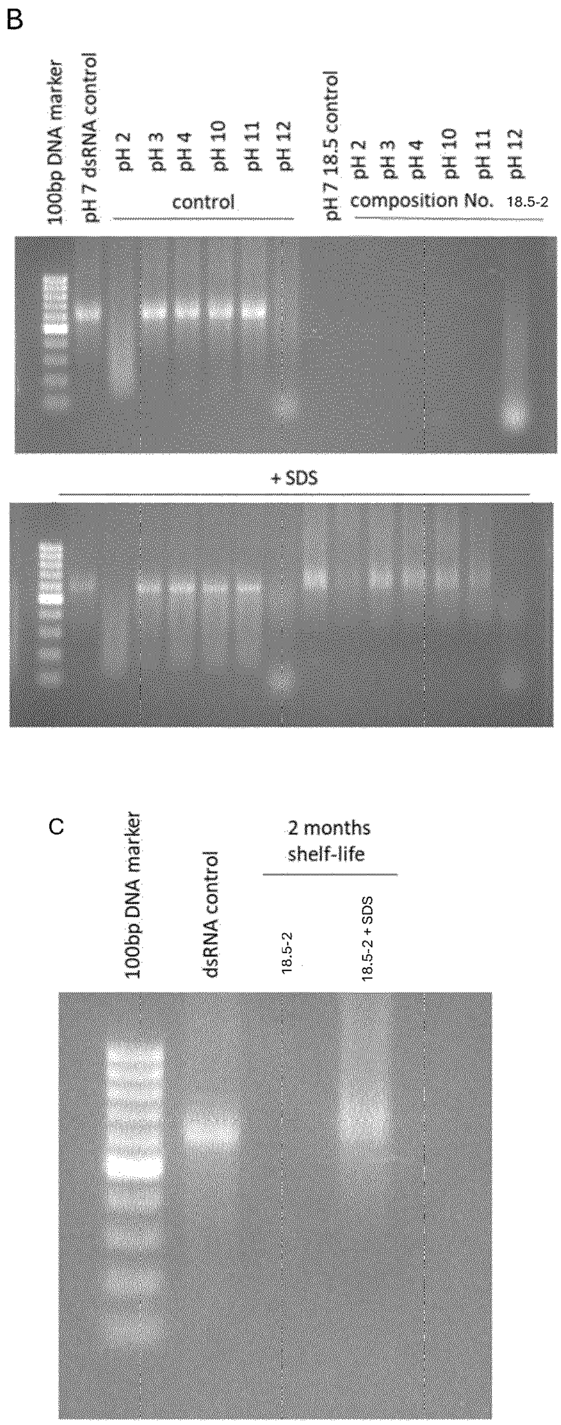

FIGURE 8

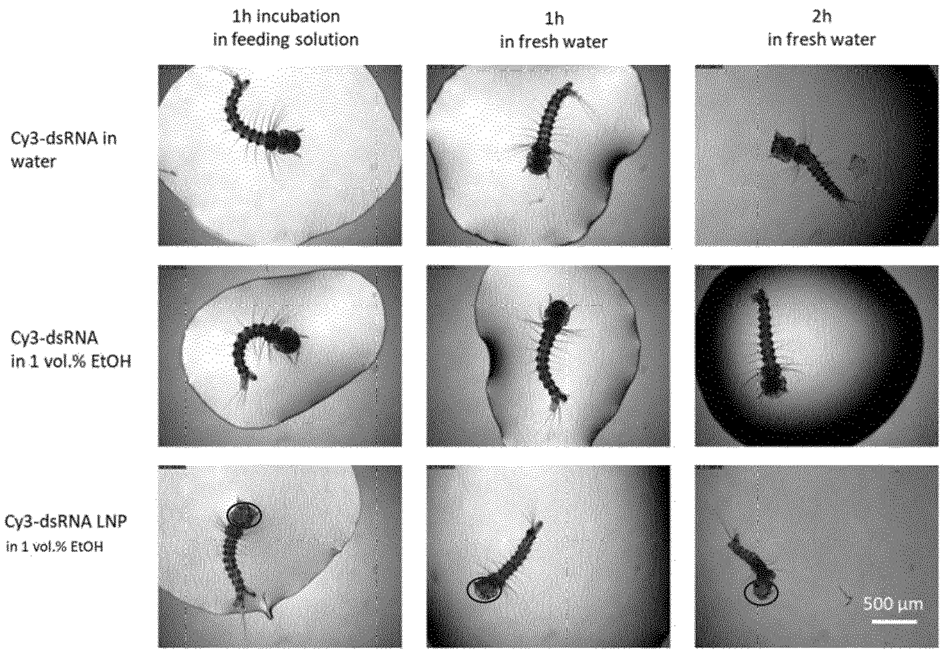
1h incubation
in feeding solution
1h
in fresh water
2h
in fresh water
Cy3-dsRNA in
water
Cy3-dsRNA
in 1 vol.% EtOH
Cy3-dsRNA LNP
in 1 vol.% EtOH
500 µm

Europäisches Patentamt
European Patent Office
Office européen des brevets

# EUROPEAN SEARCH REPORT

Application Number
EP 24 22 0250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2005/007196 A2 (PROTIVA BIOTHERAPEUTICS INC [CA]; MACLACHLAN IAN [CA] ET AL.) 27 January 2005 (2005-01-27)<br>* claims 1-8, 14, 16-17, 54, 56-57 *<br>* page 10, paragraph 53 - page 11, paragraph 57 *<br>* page 14, paragraph 65 *<br>* page 21, paragraph 89 - page 22, paragraph 91 *<br>* page 27, paragraph 108 *<br>* page 31, paragraph 120 - page 32 *<br>* page 35, paragraph 130 - paragraph 133 *<br>----- | 1-15 | INV.<br>A01P7/00<br>A61K31/713<br>C12N15/11<br>A01N63/60 |
| Y | EP 1 774 962 A1 (IND TECH RES INST [TW]) 18 April 2007 (2007-04-18)<br>* claims 1-9, 12 *<br>* page 3, column 3, paragraphs 13-14, 17 *<br>----- | 1-15 | |
| Y | WO 2008/103276 A2 (MERCK & CO INC [US]; JADHAV VASANT [US] ET AL.) 28 August 2008 (2008-08-28)<br>* claims 1-7 *<br>* page 138 - page 139 *<br>* page 277 - page 280; table IV *<br>* page 257 - page 259; example 19 *<br>----- | 1-15 | TECHNICAL FIELDS SEARCHED (IPC)<br>A61K<br>C12N<br>A01N<br>A01P |
| Y | WO 2013/126803 A1 (PROTIVA BIOTHERAPEUTICS INC [CA]) 29 August 2013 (2013-08-29)<br>* claims 14-25, 28, 30, 34 *<br>* page 23, paragraph 82 - page 24 *<br>* page 67, paragraph 210 *<br>* page 85, paragraph 276 - page 86, paragraph 277 *<br>* page 86, paragraph 280 *<br>-----<br>-/-- | 1-15 | |

The present search report has been drawn up for all claims

1

| Place of search | Date of completion of the search | Examiner |
|---|---|---|
| Munich | 7 July 2025 | Größl, Sylvester |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 22 0250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/172045 A1 (UNIV BRITISH COLUMBIA [CA]) 23 October 2014 (2014-10-23)<br>* claims 1-14, 21-26 *<br>* page 17, line 6 - page 18, line 28 *<br>* page 22, line 14 - line 19 *<br>* page 36, line 3 - line 19 *<br>* page 36, line 27 - page 37, line 5 *<br>* page 43, line 8 - page 45, line 6; example 1 *<br>----- | 1-15 | |
| Y | WO 2022/216787 A2 (HOPE CITY [US]) 13 October 2022 (2022-10-13)<br>* claims 1-16, 22-24 *<br>* page 7, paragraph 27 - page 9, paragraph 32 *<br>* page 58, paragraph 190 *<br>----- | 1-15 | |
| Y | WO 2024/010330 A1 (GREEN CROSS CORP [KR]) 11 January 2024 (2024-01-11)<br>* claims 1-16 *<br>* page 7, paragraph 75 - page 8, paragraph 76 *<br>* page 8, paragraph 78 - page 9, paragraph 79 *<br>* page 9, paragraphs 81-82, 84-85 *<br>* page 12 - page 14; table 1 *<br>----- | 1-15 | TECHNICAL FIELDS SEARCHED (IPC) |
| Y | WO 03/004644 A1 (COMMW SCIENT IND RES ORG [AU]; WHYARD STEVEN [AU] ET AL.) 16 January 2003 (2003-01-16)<br>* claims 1-8, 12-14, 17-18, 28 *<br>* page 13, line 16 - page 14, line 31 *<br>* page 33, line 8 - line 12; table 4 *<br>* page 36, line 9 - page 37, line 21; table 7 *<br>-----<br>-/-- | 1-15 | |

1

The present search report has been drawn up for all claims

| Place of search | Date of completion of the search | Examiner |
|---|---|---|
| Munich | 7 July 2025 | Größl, Sylvester |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 22 0250

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VALERIA BONINA ET AL: "The use of RNA interference for the management of arthropod pests in livestock farms", MEDICAL AND VETERINARY ENTOMOLOGY, BLACKWELL SCIENTIFIC PUBL., OXFORD, GB, vol. 37, no. 4, 4 July 2023 (2023-07-04), pages 631-646, XP072528580, ISSN: 0269-283X, DOI: 10.1111/MVE.12677 title; * abstract * * ch. "DOMAIN OF HTE REVIEW"; page 633 * ----- | 1-15 | |

TECHNICAL FIELDS SEARCHED (IPC)

The present search report has been drawn up for all claims

1

| Place of search | Date of completion of the search | Examiner |
|---|---|---|
| Munich | 7 July 2025 | Größl, Sylvester |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO. EP 24 22 0250

This annex lists the patent family members relating to the patent documents cited in the above-mentioned European search report. The members are as contained in the European Patent Office EDP file on
The European Patent Office is in no way liable for these particulars which are merely given for the purpose of information.

07-07-2025

| Patent document cited in search report | Publication date | Patent family member(s) | Publication date |
|---|---|---|---|
| WO 2005007196 A2 | 27-01-2005 | AU 2004257373 A1 | 27-01-2005 |
| | | AU 2011201463 A1 | 21-04-2011 |
| | | CA 2532228 A1 | 27-01-2005 |
| | | CN 101291653 A | 22-10-2008 |
| | | EP 1648519 A2 | 26-04-2006 |
| | | EP 2567693 A1 | 13-03-2013 |
| | | ES 2559828 T3 | 16-02-2016 |
| | | HK 1123751 A1 | 26-06-2009 |
| | | IL 173052 A | 31-03-2015 |
| | | JP 4951338 B2 | 13-06-2012 |
| | | JP 5784914 B2 | 24-09-2015 |
| | | JP 5977394 B2 | 24-08-2016 |
| | | JP 2007528863 A | 18-10-2007 |
| | | JP 2011126892 A | 30-06-2011 |
| | | JP 2013136645 A | 11-07-2013 |
| | | JP 2015143273 A | 06-08-2015 |
| | | KR 20060028655 A | 30-03-2006 |
| | | NZ 544637 A | 30-04-2010 |
| | | SG 190613 A1 | 28-06-2013 |
| | | US 2005064595 A1 | 24-03-2005 |
| | | US 2006240093 A1 | 26-10-2006 |
| | | US 2012058188 A1 | 08-03-2012 |
| | | WO 2005007196 A2 | 27-01-2005 |
| EP 1774962 A1 | 18-04-2007 | CN 1947702 A | 18-04-2007 |
| | | EP 1774962 A1 | 18-04-2007 |
| | | JP 5264053 B2 | 14-08-2013 |
| | | JP 2007106740 A | 26-04-2007 |
| | | TW 200714292 A | 16-04-2007 |
| | | US 2007087045 A1 | 19-04-2007 |
| WO 2008103276 A2 | 28-08-2008 | AU 2008219165 A1 | 28-08-2008 |
| | | CA 2689042 A1 | 28-08-2008 |
| | | EP 2131848 A2 | 16-12-2009 |
| | | JP 2010519203 A | 03-06-2010 |
| | | US 2010015218 A1 | 21-01-2010 |
| | | WO 2008103276 A2 | 28-08-2008 |
| WO 2013126803 A1 | 29-08-2013 | AU 2013222179 A1 | 09-10-2014 |
| | | BR 112014020824 A2 | 20-06-2017 |
| | | CA 2865412 A1 | 29-08-2013 |
| | | CN 104321304 A | 28-01-2015 |
| | | CN 110003030 A | 12-07-2019 |
| | | CY 1121232 T1 | 29-05-2020 |
| | | DK 2817287 T3 | 02-01-2019 |
| | | EP 2817287 A1 | 31-12-2014 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 24 22 0250

This annex lists the patent family members relating to the patent documents cited in the above-mentioned European search report. The members are as contained in the European Patent Office EDP file on
The European Patent Office is in no way liable for these particulars which are merely given for the purpose of information.

07-07-2025

| Patent document cited in search report | Publication date | Patent family member(s) | Publication date |
|---|---|---|---|
| | | EP 3473611 A1 | 24-04-2019 |
| | | EP 3988104 A1 | 27-04-2022 |
| | | ES 2702874 T3 | 06-03-2019 |
| | | ES 2898912 T3 | 09-03-2022 |
| | | HK 1202855 A1 | 09-10-2015 |
| | | HR P20182156 T1 | 22-02-2019 |
| | | HU E041494 T2 | 28-05-2019 |
| | | IL 276924 A | 29-10-2020 |
| | | IL 290931 A | 01-04-2022 |
| | | JP 6275655 B2 | 07-02-2018 |
| | | JP 6789918 B2 | 25-11-2020 |
| | | JP 7110297 B2 | 01-08-2022 |
| | | JP 2015509505 A | 30-03-2015 |
| | | JP 2018039844 A | 15-03-2018 |
| | | JP 2021014462 A | 12-02-2021 |
| | | JP 2022140533 A | 26-09-2022 |
| | | KR 20140129263 A | 06-11-2014 |
| | | KR 20200016396 A | 14-02-2020 |
| | | KR 20210041135 A | 14-04-2021 |
| | | KR 20220045089 A | 12-04-2022 |
| | | LT 2817287 T | 27-12-2018 |
| | | MX 356904 B | 07-06-2018 |
| | | MX 392812 B | 24-03-2025 |
| | | NZ 700075 A | 27-05-2016 |
| | | PH 12014501901 A1 | 24-11-2014 |
| | | PL 2817287 T3 | 31-10-2019 |
| | | PT 2817287 T | 28-12-2018 |
| | | RU 2014138476 A | 10-04-2016 |
| | | RU 2020111326 A | 29-04-2020 |
| | | SG 11201405157P A | 30-10-2014 |
| | | SI 2817287 T1 | 28-02-2019 |
| | | UA 120026 C2 | 25-09-2019 |
| | | US 2015064242 A1 | 05-03-2015 |
| | | US 2017007702 A1 | 12-01-2017 |
| | | US 2020306378 A1 | 01-10-2020 |
| | | US 2023109183 A1 | 06-04-2023 |
| | | WO 2013126803 A1 | 29-08-2013 |
| | | ZA 201406896 B | 25-05-2016 |
| WO 2014172045 A1 | 23-10-2014 | CA 2906732 A1 | 23-10-2014 |
| | | CN 105143456 A | 09-12-2015 |
| | | EP 2971013 A1 | 20-01-2016 |
| | | JP 6605446 B2 | 13-11-2019 |
| | | JP 2016515815 A | 02-06-2016 |
| | | US 2016022580 A1 | 28-01-2016 |
| | | US 2019307689 A1 | 10-10-2019 |

EPO FORM P0459

For more details about this annex : see Official Journal of the European Patent Office, No. 12/82

# ANNEX TO THE EUROPEAN SEARCH REPORT ON EUROPEAN PATENT APPLICATION NO. EP 24 22 0250

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2025

| Patent document cited in search report | Publication date | Patent family member(s) | Publication date |
|---|---|---|---|
| | | WO 2014172045 A1 | 23-10-2014 |
| WO 2022216787 A2 | 13-10-2022 | US 2024229034 A1 | 11-07-2024 |
| | | WO 2022216787 A2 | 13-10-2022 |
| WO 2024010330 A1 | 11-01-2024 | AU 2023304925 A1 | 23-01-2025 |
| | | CN 119317431 A | 14-01-2025 |
| | | EP 4551200 A1 | 14-05-2025 |
| | | KR 20240006456 A | 15-01-2024 |
| | | TW 202402304 A | 16-01-2024 |
| | | WO 2024010330 A1 | 11-01-2024 |
| WO 03004644 A1 | 16-01-2003 | CA 2455490 A1 | 16-01-2003 |
| | | DK 1414959 T3 | 15-08-2016 |
| | | EP 1414959 A1 | 06-05-2004 |
| | | EP 2333061 A1 | 15-06-2011 |
| | | ES 2650214 T3 | 17-01-2018 |
| | | JP 4330987 B2 | 16-09-2009 |
| | | JP 2004532653 A | 28-10-2004 |
| | | JP 2009185044 A | 20-08-2009 |
| | | NZ 530969 A | 30-09-2005 |
| | | US 2005095199 A1 | 05-05-2005 |
| | | US 2012041053 A1 | 16-02-2012 |
| | | US 2012309813 A1 | 06-12-2012 |
| | | US 2013237586 A1 | 12-09-2013 |
| | | US 2015047063 A1 | 12-02-2015 |
| | | US 2015275214 A1 | 01-10-2015 |
| | | US 2017260528 A1 | 14-09-2017 |
| | | US 2019316128 A1 | 17-10-2019 |
| | | WO 03004644 A1 | 16-01-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VERMA** ; **ECKSTEIN**. *Annu. Rev. Biochem.*, 1998, vol. 67, 99-134